# FASCICULE DE BREVET EUROPEEN

(11) **EP 4 036 932 B1**
(45) Date de publication et mention de la délivrance du brevet: **30.09.2026**
(21) Numéro de dépôt: 22154140.2
(22) Date de dépôt: 31.01.2022
(51) Int. Cl.: G16H 50/50

(54) **SYSTÈME DE DÉTERMINATION D'UN SCÉNARIO DE REPOS EN VOL D'UN ÉQUIPAGE D AÉRONEF**
SYSTEM ZUR BESTIMMUNG EINES SZENARIOS FÜR DIE RUHEZEIT EINER FLUGZEUGBESATZUNG WÄHREND DES FLUGS
SYSTEM FOR DETERMINING AN IN-FLIGHT REST SCENARIO FOR AN AIRCRAFT CREW

(30) Priorité: 01.02.2021 FR 2100938
(43) Date de publication de la demande: 03.08.2022
(73) Titulaire: Dassault Aviation, 92210 Saint-Cloud (FR)
(72) Inventeur: SALMON-LEGAGNEUR, François, 92214 Saint-Cloud (FR); DENJEAN, Jean-Christophe, 33700 Merignac (FR); LIGIER, Valentin, 92214 Saint-Cloud (FR); MICHON, Astrid, 92214 Saint-Cloud (FR)
(74) Mandataire: Lavoix

(56) Documents cités:
- US-A1- 2005 154 634
- US-A1- 2011 071 873
- US-A1- 2020 290 740
- SUN RUISHAN ET AL: "Study on a Model of Flight Fatigue Dynamic Risk Index", 22 June 2014, ADVANCES IN BIOMETRICS : INTERNATIONAL CONFERENCE, ICB 2007, SEOUL, KOREA, AUGUST 27 - 29, 2007 ; PROCEEDINGS; [LECTURE NOTES IN COMPUTER SCIENCE; LECT.NOTES COMPUTER], SPRINGER, BERLIN, HEIDELBERG, PAGE(S) 375 - 386, ISBN: 978-3-540-74549-5, XP047295807

## Description

La présente invention concerne un système de détermination d'un scénario de repos en vol d'un équipage d'aéronef.

En cours de vol, il est connu que la fatigue et l'augmentation du temps d'éveil affecte les performances d'un individu. Cette augmentation aggrave la probabilité que des erreurs soient commises et que des incidents arrivent. Afin de remédier à cette tendance, les pilotes peuvent profiter de périodes de repos pendant la croisière de certains vols (par exemple, lors des vols mettant en œuvre un équipage surnuméraire). Cela permet aux pilotes d'alterner pendant la croisière entre les périodes de service cockpit et les périodes de repos.

Habituellement, en opération, l'équipage détermine à l'amiable la répartition des plages de repos entre les pilotes pendant la croisière. Le capitaine reste responsable de cette organisation, et peut aussi donner le choix du créneau de repos au pilote qui sera chargé de la descente et de l'atterrissage de l'aéronef en fin de vol.

De plus, si un des pilotes de l'équipage présente un état de fatigue trop avancé au cours du vol, la réglementation aérienne peut autoriser le pilote à faire une sieste qui n'était pas planifiée initialement.

Cependant, ces approches habituelles ne sont pas satisfaisantes. En effet, elles sont approximatives et n'assurent pas systématiquement l'optimisation du niveau de fatigue des pilotes de l'équipage pendant le vol. Ces approches connues peuvent donc faire courir un risque quant à la sécurité de l'aéronef pendant le vol.

Le document US 2011/071873 décrit un système propre à analyser la fatigue d'un équipage d'aéronef. Le document US 2005/154634 décrit une méthode permettant d'évaluer la fatigue pour un membre d'équipage d'aéronef. Le document US 2020/290740 décrit un système de détermination de l'état de fatigue de membres d'un équipage d'aéronef. Enfin, l'article (D4) de Sun Ruishan et al. intitulé « Study on a Model of Flight Fatigue Dynamic Risk Index » décrit différents modèles biomathématiques de fatigue.

Un but de l'invention est donc de fournir un système permettant d'améliorer la sécurité de l'aéronef en minimisant la fatigue des pilotes pendant le vol.

À cet effet, l'invention a pour objet un système de détermination d'un scénario de repos en vol d'un équipage d'aéronef selon la revendication 1.

Le système selon l'invention permet l'optimisation du repos en vol d'un équipage d'aéronef et la minimisation de son état de fatigue au cours de l'opération.

Le système selon l'invention est de préférence selon l'une quelconque des revendications 2 à 13.

Le système selon l'invention peut comprendre l'une ou plusieurs des caractéristiques suivantes, prise(s) isolément ou suivant toute combinaison techniquement possible :
- ladite action de modification du scénario de repos affiché comprend une modification d'une position temporelle d'au moins une des plages de repos du scénario et/ou l'ajout d'une nouvelle plage de repos, et/ou la suppression d'une des plages de repos ;
- les entrées de contexte et de contraintes de vol peuvent comprendre, pour chaque pilote, un paramètre de repos après-vol représentatif d'une possibilité de repos pour le pilote après atterrissage de l'aéronef et avant tout autre décollage, la relation d'ordre étant établie aussi en fonction desdits paramètres de repos après-vol ;
- le module de détermination est configuré pour déterminer le scénario de repos en amont d'un vol et/ou pendant un vol de l'aéronef ;
- le pas de discrétisation est déterminé par le module de détermination à partir de paramètres de contraintes de calcul, les paramètres de contraintes de calcul comprenant de préférence une durée maximale de calcul, une durée estimée de calcul par chronologie, un nombre de plage(s) de repos à prévoir, une durée de chaque plage de repos et une durée de croisière disponible ;
- la durée de croisière disponible correspond à l'écart entre un instant calculé de sommet de montée de l'aéronef et un instant calculé de début de descente de l'aéronef, ou l'écart entre un instant courant du vol et un instant calculé de début de descente de l'aéronef ; et
- le module de gestion d'affichage est configuré pour afficher en outre au moins un scénario de repos alternatif, le scénario de repos alternatif comprenant la chronologie compatible ayant le deuxième meilleur classement, selon la relation d'ordre.

L'invention a aussi pour objet un aéronef comprenant le système de détermination d'un scénario de repos en vol tel que décrit plus haut.

L'aéronef peut comprendre l'une ou plusieurs des caractéristiques suivantes, prise(s) isolément ou suivant toute combinaison techniquement possible :
- l'aéronef comprend au moins un système embarqué, le système de détermination étant connecté au système embarqué, le système de détermination étant configuré pour envoyer au système embarqué un signal représentatif du scénario de repos déterminé ;
- le système embarqué est une imprimante, ou un système avionique ; et
- le système embarqué comprend un dispositif de réveil d'un pilote au repos et un module de réveil, connecté audit système de détermination, configuré pour actionner le dispositif de réveil en fonction du scénario de repos déterminé.

L'invention a également pour objet une méthode de détermination d'un scénario de repos en vol d'un équipage d'aéronef selon la revendication 14.

L'invention sera mieux comprise à la lecture de la description qui va suivre, donnée uniquement à titre d'exemple, et faite en se référant aux dessins annexés, sur lesquels :
[Fig 1] la figure 1 est un organigramme schématique d'un système de détermination selon un exemple de réalisation de l'invention ;
[Fig 2] [Fig 3] les figures 2 et 3 sont respectivement des exemples d'une fenêtre d'acquisition d'entrées et d'une fenêtre de résultats affichées par le système de détermination de la figure 1 ; et
[Fig 4] la figure 4 est un organigramme schématique d'une méthode de détermination selon un exemple de réalisation de l'invention.

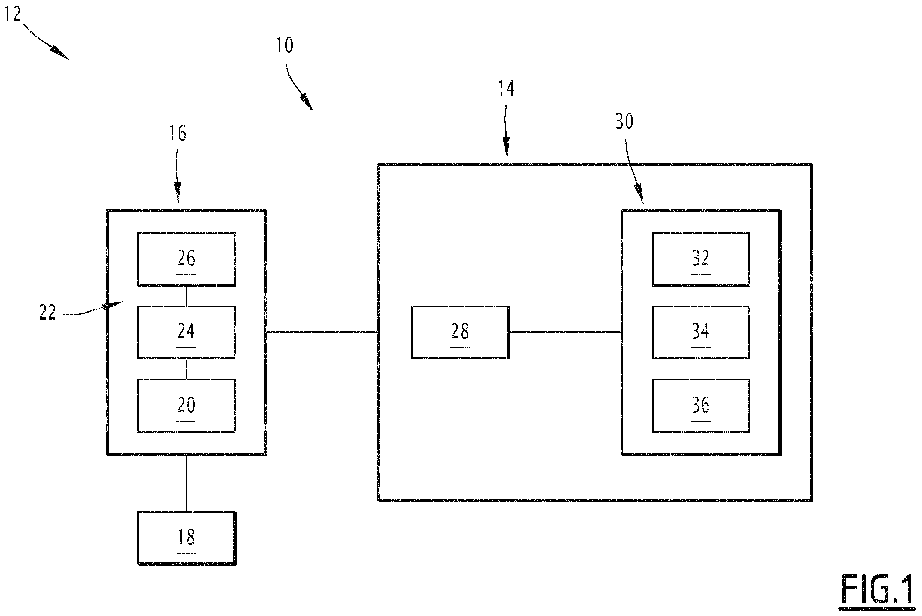

Un système 10 de détermination d'un scénario de repos en vol d'un équipage d'aéronef 12 selon un mode de réalisation de l'invention est illustré sur la Figure 1.

L'aéronef 12 comprend au moins un système embarqué, tel qu'une imprimante et/ou un système avionique.

L'équipage comprend au moins deux pilotes propres à piloter l'aéronef 12 pendant un vol de l'aéronef 12.

En particulier, l'équipage comprend plus de pilotes que le strict nécessaire pour piloter l'aéronef 12, et lors d'un vol, au moins un desdits pilotes peut être au repos pendant que le ou les autres pilotes sont en service selon les règles définies par chaque compagnie aérienne.

Ici et par la suite, on entend par « pilote en service », un pilote qui assure les activités professionnelles liées à son poste pendant une plage de service donnée. Par exemple, un pilote en service est aux commandes de l'aéronef 12 dans le cockpit.

Le système 10 est par exemple compris dans l'aéronef 12.

Le système 10 comporte une unité de traitement 14, un dispositif 16 d'affichage et une interface homme machine 18.

Le système 10 est de préférence connecté audit système embarqué de l'aéronef 12.

Le dispositif 16 d'affichage est de préférence placé dans le cockpit de l'aéronef 12.

Le dispositif 16 d'affichage comprend un écran 20.

Le dispositif 16 d'affichage comprend aussi des moyens 22 de traitement de l'information graphique, par exemple un processeur graphique 24 et une mémoire graphique 26 associée.

Le processeur graphique 24 est adapté pour traiter l'information graphique stockée dans la mémoire graphique 26 et réaliser l'affichage sur l'écran 20 de cette information ou d'une représentation de celle-ci.

L'interface homme machine 18 est propre à permettre à l'équipage d'interagir avec l'unité de traitement 14 et/ou avec le dispositif 16 d'affichage, comme décrit ci-après.

L'interface homme machine 18 comprend un dispositif de commande par exemple tactile, configuré pour détecter la position d'un ou plusieurs organe(s), appelés par la suite organes de commande, sur une surface de ce dispositif de commande. De manière connue, ces organes de commande peuvent être un stylet ou les doigts d'un opérateur.

Dans la suite de la description, on considèrera un mode de réalisation préféré dans lequel ce dispositif de commande tactile et l'écran 20 présentent une forme commune, sous la forme d'un écran tactile. Ainsi, l'interface homme machine 18 est configurée pour détecter la position d'un ou plusieurs organe(s) de commande, sur la surface de l'écran 20.

L'unité de traitement 14 est adaptée pour exécuter des applications nécessaires au fonctionnement du système de détermination 10.

A cette fin, l'unité de traitement 14 comprend un processeur 28 et au moins une mémoire 30.

Le processeur 28 est adapté pour exécuter des applications contenues dans la mémoire 30, de préférence un système d'exploitation permettant le fonctionnement classique d'un système informatique.

L'unité de traitement 14 comprend ainsi au moins un module 32 d'acquisition d'entrées de contexte et de contraintes de vol et un module 34 de détermination d'au moins un scénario de repos de l'équipage pour le vol de l'aéronef 12 à partir desdites entrées acquises.

L'unité de traitement 14 comprend aussi un module 36 de gestion d'affichage configuré pour afficher le scénario de repos déterminé sur l'écran 20 du dispositif d'affichage 16.

Lesdits modules 32, 34, 36 sont par exemple stockés dans la mémoire 30 et sont propres à être exécutés par le processeur 28 pour mettre en œuvre les fonctions décrites ci-après. En variante, chaque module 32, 34, 36 est mis en œuvre sous forme de composants logiques programmables ou de circuits intégrés dédiés, destinés à réaliser les fonctions décrites ci-après.

Dans un mode de réalisation préféré, le module d'acquisition 32 est propre à acquérir les entrées par l'intermédiaire de l'interface homme-machine 18, par interrogation de la mémoire 30 et/ou par l'intermédiaire de capteurs de l'aéronef 12.

Une fois les entrées acquises, le module d'acquisition 32 est par exemple propre à les stocker dans la mémoire 30.

Différents exemples d'entrées de contexte et de contraintes de vol propres à être acquises par le module d'acquisition 32 vont maintenant être décrits.

Les entrées de contexte et de contraintes de vol comprennent par exemple au moins le nombre de pilotes de l'équipage et, de préférence, des informations caractéristiques associées à chaque pilote.

Les informations caractéristiques sont stockées dans la mémoire 30 et/ou sont acquises en étant renseignées par un membre de l'équipage, par l'intermédiaire de l'interface homme-machine 18.

Les informations caractéristiques peuvent comprendre par exemple l'âge du pilote et le statut hiérarchique du pilote au sein de l'équipage. Un des pilotes a par exemple ainsi le statut de capitaine de l'équipage, comme statut hiérarchique.

Les informations caractéristiques comprennent par exemple aussi un identifiant du pilote, tel que le nom du pilote.

Ainsi, le système de détermination 10 est flexible à la composition de l'équipage.

Les entrées de contexte et de contraintes de vol comprennent aussi une durée de plage de repos prédéterminée.

La durée de plage de repos est par exemple acquise en étant renseignée par un membre de l'équipage par l'intermédiaire de l'interface homme-machine 18.

La durée de plage de repos est par exemple égale pour chaque plage de repos qui est déterminée.

La durée de plage de repos comprend notamment une durée de sommeil autorisé suivie d'une durée de transition de poste prédéterminée. Comme expliqué plus en détails ci-dessous, la durée de plage de repos est par exemple renseignée manuellement par un des pilotes.

La durée de transition de poste est définie comme une période, avant la reprise de service, au cours de laquelle le pilote est préparé pour la reprise du service, par exemple par un autre pilote en service.

La durée de sommeil autorisé et la durée de transition de poste sont par exemple acquises en étant renseignées par un membre de l'équipage, par l'intermédiaire de l'interface homme-machine 18.

Les entrées de contexte et de contraintes de vol comprennent aussi un nombre de plage(s) de repos nécessaire(s) à prévoir pour l'équipage.

Le nombre de plage(s) de repos nécessaire(s) à prévoir pour l'équipage est par exemple déterminé en fonction d'un nombre de pilote(s) pour lequel/lesquels une plage de repos est à prévoir et d'un nombre de plage(s) de repos pour chaque pilote.

Le nombre de plage(s) de repos est par exemple acquis en étant renseigné par un membre de l'équipage par l'intermédiaire de l'interface homme-machine 18.

Le nombre de plage(s) de repos pour chaque pilote n'est pas forcément le même pour tous les pilotes de l'équipage.

Ceci est par exemple le cas, lorsque la détermination du scénario de repos est réalisée en cours de vol et qu'au moins un des pilotes a déjà réalisé au moins une plage de repos. Le nombre de plage(s) de repos à prévoir pour au moins un des pilotes peut dans ce cas par exemple être nul, le pilote n'étant alors plus autorisé à se reposer pendant le reste du vol.

Les entrées de contexte et de contraintes de vol comprennent aussi des informations relatives au vol.

La mémoire 30 stocke par exemple une pluralité de vols et des informations relatives à chaque vol stocké.

Chaque vol comprend au moins une phase de montée de décollage, une phase de croisière, et une phase de descente d'atterrissage. La phase de croisière est en particulier définie comme la phase du vol entre un sommet de montée et un début de descente. La phase de croisière comprend au moins une altitude de croisière (appelée aussi niveau de vol).

Le sommet de montée, également appelé TOC (pour l'acronyme anglais de « top of climb »), est la transition calculée de la phase de montée d'un vol à la phase de croisière, le point auquel la montée prévue à une altitude de croisière est terminée.

Le début de descente, également appelé TOD (pour l'acronyme anglais de « top of descent »), est la transition calculée de la phase de croisière d'un vol à la phase de descente, ou le point auquel la descente prévue à une altitude d'approche finale est amorcée.

Avantageusement, les informations relatives au vol de l'aéronef 12 comprennent un instant calculé de sommet de montée et un instant calculé de début de descente de l'aéronef 12 pour le vol.

Les informations relatives au vol de l'aéronef 12 comprennent aussi au moins un instant de décollage calculé et un instant d'atterrissage calculé.

Chacun de ces instants calculés est de préférence calculé initialement, i.e. avant le vol, en fonction d'un plan de vol de l'aéronef 12 stocké dans la mémoire 30.

Chacun de ces instants calculés est de préférence mis à jour à plusieurs moments lors du vol de l'aéronef 12 en fonction de la position courante de l'aéronef 12 à ce moment, des capacités de l'aéronef 12 et des conditions météorologiques.

Les entrées de contexte et de contraintes de vol comprennent aussi, parmi les informations relatives au vol de l'aéronef 12, une durée de croisière disponible.

Comme indiqué ci-après, le module de détermination 34 est configuré pour être déclenché et déterminer le scénario de repos en amont du vol et/ou pendant le vol de l'aéronef 12.

Ainsi, la durée de croisière disponible correspond par exemple à l'écart entre l'instant calculé de sommet de montée de l'aéronef 12 et l'instant calculé de début de descente de l'aéronef 12, lorsqu'on se situe avant la phase de croisière. Alternativement, la durée de croisière disponible correspond par exemple à l'écart entre un instant courant du vol et l'instant calculé de début de descente de l'aéronef 12, lorsque l'instant courant a lieu en cours de phase de croisière.

D'autres informations relatives au vol de l'aéronef 12 comprennent aussi par exemple des événements destinés à se produire lors du vol. Les événements comprennent par exemple :
- des survols par l'aéronef 12 de points de passage prédéfinis, conformément au plan de vol,
- des communications radios prévues entre l'équipage et une autorité de contrôle extérieure,
- l'initiation de procédures, par exemple des procédures de changement de niveau de vol,
- la survenue d'aléas, notamment climatiques, par exemple la traversée de zones de vent fort, orageuses, ou de conditions givrantes,
- des actions spécifiques destinées à être réalisées par au moins un des pilotes de l'équipage, ces actions étant destinées à être réalisées à des instants fixés lors du vol ou lors du survol par l'aéronef 12 de points de passage fixés, et peuvent comprendre des tâches destinées à être effectuées par un des pilotes de l'équipage à titre personnel.

A chacun des événements sont associées, dans la mémoire 30, des informations relatives à cet événement. Ces informations comprennent par exemple un instant de réalisation fixé ou prévu de l'événement. Cet instant prévu est déterminé initialement, i.e. avant le vol, en fonction du plan de vol de l'aéronef 12, et est de préférence mis à jour à plusieurs moments lors du vol de l'aéronef 12 en fonction de la position de l'aéronef 12 à ce moment, des capacités de l'aéronef 12 et des conditions météorologiques.

Les entrées de contexte et de contraintes de vol comprennent au moins une contrainte de pilotage par l'équipage de l'aéronef 12. Avantageusement, les entrées comprennent une pluralité de contraintes de pilotage par l'équipage de l'aéronef 12.

Les contraintes contraignent en particulier le pilotage de l'aéronef 12 qui est fait par l'équipage.

Chaque contrainte de pilotage est par exemple acquise en étant renseignée par un membre de l'équipage par l'intermédiaire de l'interface homme-machine 18 et/ou stockée dans la mémoire 30.

De préférence, une des contraintes de pilotage est un nombre minimum requis de pilote(s) en service à chaque instant de la croisière.

Ce nombre minimum requis reflète dans quelle mesure les plages de repos de différents pilotes peuvent se superposer. Par exemple, dans le cas d'un nombre de pilotes au moins égal à trois et d'un nombre minimum requis égal à 1, il est possible de superposer des plages de repos.

Par « se superposer », on entend ici qu'une première plage de repos se superpose à une deuxième autre plage de repos, si au moins partie de la première plage de repos a lieu au même moment qu'au moins une partie de la deuxième plage de repos.

Avantageusement, une des contraintes de pilotage est au moins une zone temporelle prédéterminée de vol nécessitant un nombre prédéterminé de pilotes en service.

La zone temporelle est définie entre un instant de début et un instant de fin.

Le nombre prédéterminé est par exemple la totalité des pilotes de l'équipage. Chacun des pilotes de l'équipage doit alors être en service durant la zone temporelle. Il s'agit notamment alors d'une « no-sleep » zone.

Ladite zone temporelle prédéterminée est par exemple une zone temporelle de survol d'une zone géographique prédéfinie, par exemple d'un pays prédéfini.

Le système de détermination 10 est ainsi propre à prendre en compte des contraintes géopolitiques de survol de territoire.

De préférence, une des contraintes de pilotage est une zone temporelle de vol nécessitant un pilote prédéterminé en service parmi les pilotes de l'équipage. Ledit pilote prédéterminé doit alors être en service durant ladite zone temporelle.

Ledit pilote prédéterminé est par exemple le capitaine.

Avantageusement, une des contraintes de pilotage est un paramètre représentatif d'une tolérance à un contexte météorologique courant de vol.

Le paramètre représentatif de cette tolérance est par exemple acquis en étant renseigné par un membre de l'équipage par l'intermédiaire de l'interface homme-machine 18.

Le contexte météorologique comprend par exemple un état de condition givrante. L'état de condition givrante est une formation de glace sur des surfaces extérieures de l'aéronef 12 ou à l'intérieur d'un moteur de l'aéronef 12.

Le contexte météorologique comprend par exemple une intensité de turbulences subies par l'aéronef 12 et/ou l'occurrence d'un orage.

Le contexte météorologique courant est fourni par exemple au cours du vol par des capteurs de l'aéronef 12 et/ou par une communication avec une autorité de contrôle extérieure.

Cela permet à l'équipage de choisir dans quelle mesure le scénario de repos déterminé sera sensible aux phénomènes météorologiques. Par exemple, il est possible de définir qu'en cas d'état de condition givrante, chacun des pilotes de l'équipage doit être en service.

De préférence, les entrées de contexte et de contraintes de vol comprennent, pour chaque pilote, au moins un paramètre de fatigue avant-vol représentatif d'un état de fatigue avant-vol déclaré par le pilote.

Le paramètre de fatigue avant-vol déclaré par ledit pilote est par exemple acquis en étant renseigné par un membre de l'équipage par l'intermédiaire de l'interface homme-machine 18.

Le paramètre de fatigue avant-vol est par exemple choisi par le pilote sur une échelle d'état de fatigue ressentie subjectivement par le pilote, comprenant une pluralité d'états de fatigue.

L'échelle d'état de fatigue comprend dans un exemple de réalisation au moins un état de fatigue acceptable avant-vol, un état de fatigue mineure avant-vol, un état de fatigue modérée avant-vol et un état de fatigue significative avant-vol.

Par exemple, le paramètre est obtenu à partir de la somme d'un premier score représentatif du nombre d'heures que le pilote a dormi au cours des dernières 24 heures et à partir d'un deuxième score représentatif du nombre d'heures que le pilote a dormi au cours des dernières 48 heures. En fonction de la somme calculée, le paramètre est placé sur l'échelle d'état de fatigue.

D'autres méthodes d'évaluation du paramètre de fatigue avant-vol peuvent être choisies et sont connues de l'homme du métier.

Avantageusement, les entrées de contexte et de contraintes de vol comprennent, pour chaque pilote, un paramètre de repos après-vol représentatif d'une possibilité de repos pour le pilote après atterrissage de l'aéronef 12 et avant tout autre décollage.

Le système de détermination 10 est ainsi propre à prendre en compte les possibilités de repos ultérieures de chaque pilote de l'équipage, qui peuvent être différentes d'un pilote à l'autre.

Une fois les entrées de contexte et de contraintes de vol acquises par le module d'acquisition 32, le module de détermination 34 est configuré pour déterminer le scénario de repos de l'équipage à partir desdites entrées acquises. Cette détermination est faite à partir d'au moins une simulation par un modèle biomathématique de fatigue ayant pour variables au moins une desdites entrées acquises, comme décrit plus en détails par la suite.

Comme indiqué ci-dessus, le module de détermination 34 est configuré pour être déclenché et déterminer le scénario de repos en amont d'un vol et/ou pendant un vol de l'aéronef 12.

Le scénario de repos est avantageusement déterminé pour au moins une partie de la phase de croisière du vol.

Le scénario de repos comprend une chronologie de plage(s) de repos, chaque plage étant attribuée à un des pilotes. Le scénario de repos déterminé comprend aussi, pour chaque pilote, une ou des plages de service.

Pour chaque pilote, les plages de service du pilote sont accolées en continu à chaque plage de repos du pilote. Dans le scénario de repos déterminé, chaque pilote alterne ainsi de préférence une ou des plages de service avec le nombre de plage(s) de repos déterminé à prévoir pour ce pilote.

Pour déterminer le scénario, le module de détermination 34 est configuré pour déterminer au moins une chronologie de plage(s) de repos compatible avec chaque contrainte de pilotage acquise.

Avantageusement, avant de déterminer chaque chronologie de plage(s) de repos compatible, le module de détermination 34 est configuré pour vérifier au moins une condition de compatibilité entre au moins deux des entrées acquises.

Dans un exemple préféré de réalisation, le module 34 est ainsi configuré pour au moins vérifier une condition de compatibilité entre la durée de croisière disponible et les plages de repos à prévoir. Plus précisément, la condition de compatibilité est alors par exemple que la durée de croisière disponible est suffisamment longue pour inclure chaque plage de repos.

Si la condition de compatibilité entre entrées n'est pas vérifiée, le module de détermination 34 est de plus par exemple configuré pour modifier au moins une des entrées acquises, par exemple au moins une des entrées intervenant dans la condition de compatibilité. La modification est réalisée de sorte que la condition de compatibilité entre entrées modifiées soit respectée.

Dans l'exemple préféré, le module de détermination 34 est configuré pour réduire la durée d'au moins une des plages de repos à prévoir, si la durée de croisière disponible est trop courte pour inclure chaque plage de repos. Dans ce cas, le module de détermination 34 réduit avantageusement la durée de sommeil autorisée, en conservant la durée de transition de poste prédéterminée.

Le module de détermination 34 est aussi configuré pour générer une alerte à destination de l'équipage si au moins une condition de compatibilité entre entrées acquises n'est pas vérifiée. L'alerte est aussi par exemple représentative, le cas échéant, de chaque modification des entrées faite par le module de détermination 34. Pour ce faire, le module est de préférence configuré pour générer un signal, par exemple ici à destination du module de gestion d'affichage 36, le signal étant représentatif de la vérification de chaque condition de compatibilité. Le signal est aussi représentatif, le cas échéant, de chaque modification des entrées par le module de détermination 34.

Dans l'exemple préféré, le module de détermination 34 est configuré pour générer une alerte à destination de l'équipage, si la durée de croisière disponible est trop courte pour inclure chaque plage de repos.

Toute autre condition de compatibilité entre entrées acquises peut aussi être vérifiée de la sorte.

Dans le but de déterminer chaque chronologie qui est compatible avec chaque contrainte de pilotage acquise, le module de détermination 34 est par exemple configuré pour discrétiser la durée de croisière disponible acquise en une pluralité d'instants discrétisés.

La discrétisation est faite avec un pas de discrétisation déterminé.

Comme expliqué plus en détails par la suite, le pas de discrétisation est avantageusement déterminé dynamiquement selon les contraintes de pilotage et de contexte.

Le pas de discrétisation est par exemple déterminé par le module de détermination 34 à partir de paramètres de contraintes de calcul. Les paramètres de contraintes de calcul sont par exemple stockés dans la mémoire 30.

Les paramètres de contraintes de calcul comprennent de préférence une durée maximale de calcul, une durée estimée de calcul par chronologie, le nombre de plage(s) de repos à prévoir, la durée de chaque plage de repos et la durée de croisière disponible.

La détermination du pas de discrétisation comprend par exemple la détermination du nombre maximal de chronologies à calculer permettant de respecter la contrainte de durée maximale de calcul, à partir de la durée maximale de calcul et de la durée estimée de calcul par chronologie.

La détermination du pas de discrétisation comprend alors la détermination du nombre maximum de positions temporelles par plage permettant de respecter la contrainte de durée maximale de calcul.

La détermination dudit nombre maximum est faite par exemple en prenant la racine n-ième dudit nombre maximal de chronologies à calculer permettant de respecter la contrainte de durée maximale de calcul, où n est le nombre de plage(s) de repos à prévoir.

La détermination du pas de discrétisation comprend ensuite le calcul de la différence entre la durée de croisière disponible et la durée de chaque plage de repos, et la division de cette différence par ledit nombre maximum de positions temporelles par plage auquel est retranché 1.

La détermination du pas de discrétisation peut comprendre aussi par exemple l'arrondissement du résultat au supérieur.

Cette méthode de détermination n'est pas limitante et d'autres méthodes peuvent être utilisées par l'homme du métier. Le pas de discrétisation peut aussi alternativement être fixé indépendamment du nombre de plage(s) de repos à prévoir, de la durée de chaque plage de repos et de la durée de croisière disponible ; le pas étant alors par exemple stocké dans la mémoire 30.

Après discrétisation, le module de détermination 34 est configuré pour lister toutes les chronologies de plages de repos dans lesquelles chaque plage de repos a un instant de début correspondant à un des instants discrétisés.

Dans chaque chronologie listée, les plages de la chronologie ont toutes un instant de fin antérieur ou égal à la fin de la phase de croisière, en particulier antérieur ou égal à l'instant calculé de début de descente.

Le module est alors configuré pour vérifier, pour chaque chronologie listée, si la chronologie listée est compatible avec chaque contrainte de pilotage acquise.

Par la suite, le module de détermination 34 est configuré pour simuler et déterminer, pour chaque chronologie compatible, au moins un niveau de fatigue de chaque pilote, par le modèle biomathématique ayant pour variables au moins une desdites entrées acquises.

Toute chronologie non compatible avec au moins une des contraintes de pilotage acquises est exclue, et ne sera pas simulée par le modèle biomathématique.

Le modèle biomathématique de fatigue (ou de somnolence) est propre à quantifier un niveau de fatigue sur une échelle de fatigue prédéterminée.

Le niveau de fatigue rapportée à l'échelle de fatigue traduit les capacités neurocomportementales de l'individu, telles que par exemple l'attention, la vigilance, ou la somnolence.

En d'autres termes, au sens de l'invention, les termes « niveau de fatigue » sont synonymes de « niveau d'éveil », « niveau d'attention », « niveau de vigilance », ou encore « niveau de somnolence ».

Ainsi, lorsque le module de détermination 34 simule et détermine un niveau de fatigue, le module fournit une quantification de ce niveau de fatigue.

Le modèle biomathématique peut comprendre un ensemble d'équations ayant pour variables lesdites entrées de contexte et de contraintes de vol.

L'ensemble d'équations est ainsi propre à produire ladite quantification du niveau de fatigue.

La formule et les paramètres de chaque équation peuvent être déterminés empiriquement et sont avantageusement validés pour le domaine aéronautique.

Dans un mode de réalisation, le modèle biomathématique a en particulier comme variable chaque paramètre de fatigue avant-vol déclaré acquis.

Avantageusement, le modèle biomathématique prend en compte l'inertie du sommeil dans l'ensemble d'équations. L'inertie du sommeil est définie comme une période d'altération de la vigilance lors de la transition du sommeil à l'éveil. Cette période peut durer de quelques minutes à quelques heures.

Le modèle biomathématique peut prendre de préférence en compte une arythmie circadienne de chaque pilote (aussi appelée « jet lag » en anglais, ou « syndrome du décalage horaire »). L'arythmie circadienne est définie comme une condition physiologique qui résulte d'un vol précédent à travers plusieurs fuseaux horaires. Un tel vol décale effectivement les différentes horloges internes (rythme circadien ou cycles du sommeil) de l'individu.

Le modèle biomathématique est par exemple le SWP, pour l'anglais Sleep Wake Predictor. Ce modèle biomathématique est un modèle open source, permettant de prédire théoriquement et quantifier le niveau d'éveil d'un individu selon l'échelle KSS (pour Karolinska Sleepiness Scale en anglais) développée par le Karolinska Institute de Stockholm.

D'autres modèles biomathématiques sont connus de l'homme du métier et ne seront pas décrits plus en détails ici.

Après simulation, le module de détermination 34 est configuré pour classer chaque chronologie compatible selon une relation d'ordre établie à partir des niveaux de fatigue simulés et déterminés par le modèle.

La relation d'ordre est par exemple une formule ayant des variables, chaque variable ayant une pondération prédéterminée. Les variables comprennent au moins les niveaux de fatigue simulés.

La relation d'ordre est avantageusement choisie pour classer les chronologies en minimisant la fatigue de l'équipage pour l'atterrissage et/ou en minimisant la fatigue de chaque pilote lors de ses plages de service.

Alternativement ou en complément, la relation d'ordre est avantageusement choisie pour classer les chronologies pour faciliter l'endormissement de chaque pilote en début de chacune de ses plages de repos.

Alternativement ou en complément, la relation d'ordre est choisie pour classer les chronologies en fonction de tout autre indicateur du niveau de fatigue de chaque pilote pendant la chronologie.

Avantageusement, le module de détermination 34 est ainsi configuré pour simuler et déterminer, par le modèle biomathématique et pour chaque chronologie compatible, un niveau de fatigue maximal de chaque pilote en service pendant chaque plage de repos de chaque autre pilote et/ou un niveau de fatigue à un instant calculé de début de descente TOD de l'aéronef 12 d'au moins chaque pilote prévu en service audit instant.

Ainsi, avantageusement, la relation d'ordre est établie sur au moins un desdits niveaux de fatigue maximaux simulés et/ou sur au moins un niveau de fatigue simulé à l'instant calculé de début de descente.

Dans un exemple de réalisation non limitant, la relation d'ordre fait par exemple intervenir, dans chaque chronologie compatible, la moyenne desdits niveaux de fatigue maximaux simulés de chaque pilote en service et la moyenne desdits niveaux de fatigue au TOD.

En alternative ou en complément, la relation d'ordre est avantageusement choisie pour classer les chronologies en minimisant la fatigue d'un pilote prédéterminé au sein de l'équipage.

La relation d'ordre est par exemple ainsi établie en fonction des informations caractéristiques de chaque pilote, à savoir notamment en fonction de leurs âges et/ou de leurs statuts hiérarchiques au sein de l'équipage.

La relation d'ordre est par exemple établie aussi en fonction des paramètres de repos après-vol acquis.

Chaque pondération de la formule de la relation d'ordre est par exemple choisie pour classer les chronologies en minimisant chaque niveau de fatigue simulé à l'instant calculé de début de descente.

Le scénario de repos déterminé par le module de détermination 34 est alors le scénario comprenant la chronologie compatible ayant le meilleur classement selon ladite relation d'ordre. Une fois le scénario déterminé, le module de détermination 34 est de préférence configuré pour générer un signal, par exemple ici à destination du module de gestion d'affichage 36, le signal étant représentatif du scénario déterminé.

De préférence, le module de détermination est aussi configuré pour simuler et déterminer, par le modèle biomathématique, une évolution d'un niveau de fatigue de chaque pilote au cours de chaque plage de service du pilote dans le scénario de repos déterminé.

L'évolution est par exemple constituée par une pluralité de niveaux de fatigue au cours du temps.

Le système de détermination 10 est aussi configuré avantageusement pour envoyer au système embarqué de l'aéronef 12 un signal représentatif du scénario de repos déterminé.

Par la suite, le module de gestion d'affichage 36 est configuré pour afficher le scénario de repos déterminé sur l'écran 20 du dispositif d'affichage 16.

Après cet affichage, il est possible au pilote de relancer la détermination du scénario de repos. Cette détermination est relancée par exemple en cours de vol, par exemple en fonction de l'évolution de la mission, si la mission diffère de sa prévision avant vol.

Le module d'acquisition 32 est alors propre à acquérir de nouvelles entrées de contexte et de contraintes de vol, au moins une desdites entrées étant distincte des entrées précédemment acquises pour déterminer le scénario précédemment affiché.

Le module 34 de détermination est alors propre à déterminer au moins un nouveau scénario de repos à partir d'au moins une simulation par un modèle biomathématique de fatigue ayant pour variables au moins une desdites nouvelles entrées acquises.

Le nouveau scénario de repos est affiché sur l'écran 20 du dispositif d'affichage 16 par le module de gestion d'affichage 36.

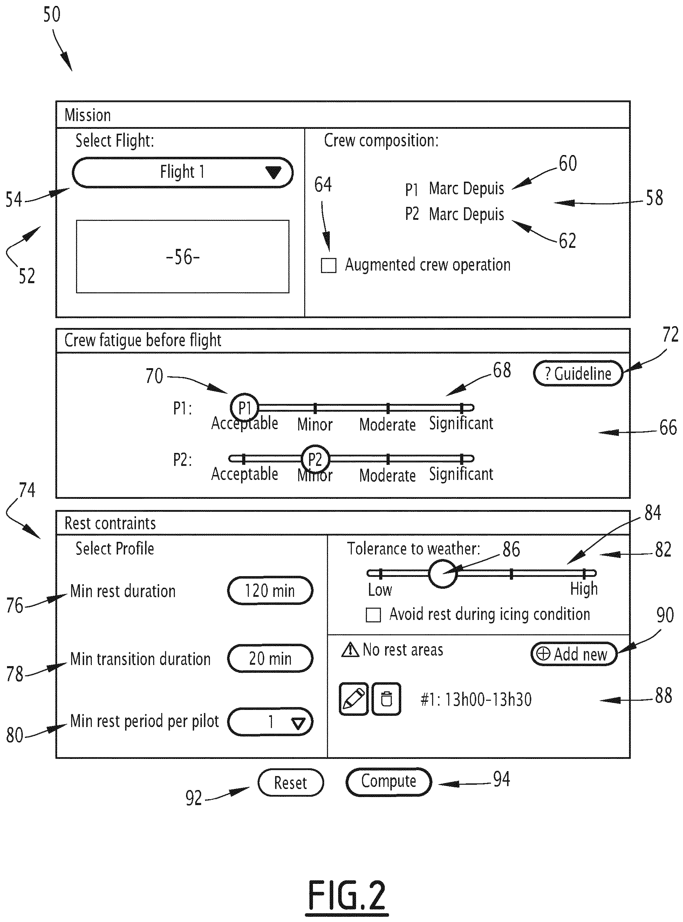

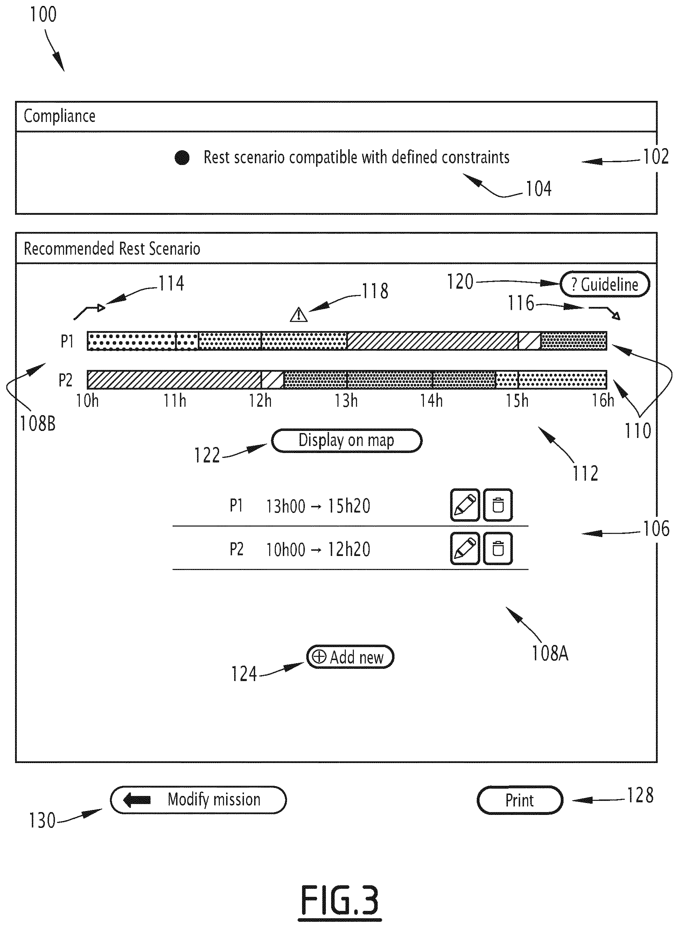

Un exemple de gestion de l'affichage sur l'écran 20 du dispositif d'affichage 16, par le module de gestion d'affichage 36, va maintenant être décrit, en référence aux figures 2 et 3.

Sur les figures 2 et 3, les annotations sont en langue anglaise dans la mesure où ces figures visent à reproduire un exemple typique d'interface graphique. Les traductions des termes anglais illustrés seront indiquées dans le texte ci-dessous.

Dans le but d'acquérir au moins une partie des entrées de contexte et de contraintes de vol, le module de gestion d'affichage 36 est configuré pour afficher, sur l'écran 20 du dispositif d'affichage 16, une fenêtre d'acquisition 50, dont un mode de réalisation non limitant est illustré sur la figure 2.

La fenêtre 50 comprend une pluralité de zones graphiques d'acquisition d'entrées.

Le module d'acquisition 32 est alors configuré pour acquérir au moins une partie des entrées de contexte et de contraintes de vol par l'actionnement d'éléments graphiques affichés dans chaque zone graphique d'acquisition.

L'actionnement est mis en œuvre par l'intermédiaire de l'interface homme-machine 18.

Dans le mode de réalisation préféré d'un écran tactile, l'actionnement est détecté par le contact desdits éléments graphiques affichés, par un ou plusieurs organe(s) de commande sur la surface de l'écran 20.

Chaque zone graphique d'acquisition d'entrées est associée à au moins une des entrées de contexte et de contraintes de vol.

Chaque zone graphique comprend de préférence une forme de cadrant.

Chaque zone graphique comprend par exemple un titre représentatif de la ou des entrées associée(s) à la zone, le titre étant disposé dans un cadrant respectif en regard de la zone. Alternativement, la zone graphique est dépourvue de titre.

La fenêtre d'acquisition 50 n'est pas limitée au mode de réalisation qui est illustré sur la figure 2 et décrit ci-dessous. Elle peut comprendre en particulier toute autre zone graphique d'acquisition d'autre(s) entrée(s).

Une des zones graphiques d'acquisition affichées est une zone 52 de sélection du vol. La zone 52 de sélection du vol est intitulée par exemple ici « Select Flight » (traduit par « Sélection du vol »).

Un membre d'équipage est propre à sélectionner un vol parmi les vols stockés dans la mémoire 30. La sélection est en particulier faite par l'intermédiaire de l'interface homme-machine 18, en actionnant un élément graphique 54 de la zone 52.

Lorsque l'élément graphique 54 est actionné, le module de gestion d'affichage 36 affiche une liste des vols stockés en mémoire 30 sous la forme d'un menu déroulant actionnable.

La zone 52 de sélection du vol affiche aussi au moins une partie 56 des informations relatives audit vol sélectionné, qui sont stockées en mémoire 30.

Une des zones graphiques d'acquisition affichées est une zone 58 de composition d'équipage. La zone 58 de composition d'équipage est ici par exemple intitulée « Crew composition » (traduit par « Composition de l'équipage »).

La zone 58 de composition d'équipage affiche la liste 60 des pilotes de l'équipage et au moins une partie 62 des informations caractéristiques de chaque pilote de l'équipage.

La zone 58 de composition d'équipage est propre à permettre l'ajout d'un nouveau pilote dans la liste 60 affichée. Pour ce faire, la zone 58 comprend de préférence une icône d'ajout 64 actionnable, intitulée par exemple ici « Augmented crew operation » (traduit par « Opérations en équipage surnuméraire »).

Dans l'exemple de réalisation illustré sur la figure 2, la zone 52 de sélection du vol et la zone 58 de composition de l'équipage sont regroupées dans une même zone graphique ayant pour titre par exemple ici « Mission ».

Une des zones graphiques d'acquisition est une zone 66 de renseignement de l'état de fatigue avant-vol. La zone 66 de renseignement de l'état de fatigue avant-vol est ici par exemple intitulée « Crew fatigue before flight » (traduit par « Fatigue de l'équipage avant-vol »).

Comme illustré sur la figure 2, la zone 66 de renseignement comprend par exemple, pour chaque pilote, une échelle d'état de fatigue 68. Par l'intermédiaire de l'échelle d'état de fatigue 68, ledit paramètre de fatigue avant-vol de chaque pilote peut être déclaré.

En particulier, l'échelle d'état de fatigue 68 est graduée, chaque graduation correspondant par exemple ici à un état de fatigue acceptable avant-vol (« Acceptable »), à un état de fatigue mineure avant-vol (« Minor »), à un état de fatigue modérée avant-vol (« Moderate ») et à un état de fatigue significative avant-vol (« Significant »).

Le paramètre est déclaré par le pilote en déplaçant un élément graphique 70 sur une des graduations de l'échelle 68 d'état de fatigue affichée, le déplacement étant réalisé par l'intermédiaire de l'interface homme-machine 18.

La zone 66 de renseignement comprend de préférence une icône d'aide 72 actionnable, par exemple intitulée ici « ? Guideline » (traduit par « ? Mode d'emploi »). Lorsque l'icône d'aide 72 est actionnée, le module de gestion d'affichage 36 est configuré pour afficher des informations d'aide et de directives au pilote pour évaluer l'état de fatigue avant-vol.

Une des zones graphiques d'acquisition est une zone 74 de sélection de repos.

La zone 74 de sélection de repos comprend par exemple un espace 76 de sélection de la durée de sommeil autorisé de chaque plage de repos, un espace 78 de sélection de la durée de transition de poste, et un espace 80 de sélection du nombre de plage(s) de repos par pilote.

Chaque sélection est respectivement faite par l'intermédiaire de l'interface homme-machine 18, en actionnant un élément graphique respectif de chaque espace 76, 78, 80.

Lorsque l'élément graphique de l'espace 76 de sélection de la durée de sommeil autorisé est actionné ou lorsque l'élément graphique de l'espace 78 de sélection de la durée de transition, le module de gestion d'affichage 36 affiche une liste de durées proposées sous la forme d'un menu déroulant actionnable ou affiche une fenêtre de saisie manuelle de la durée.

Lorsque l'élément graphique de l'espace 80 de sélection du nombre de plage(s) de repos par pilote est actionné, le module de gestion d'affichage 36 affiche une liste de nombres proposés sous la forme d'un menu déroulant actionnable ou affiche une fenêtre de saisie manuelle du nombre.

Une des zones graphiques d'acquisition est une zone 82 de tolérance aux conditions météorologiques, par exemple intitulée ici « Tolerance to weather » (traduit par « Tolérance aux phénomènes météorologiques »).

Comme illustré sur la figure 2, la zone 82 de tolérance comprend par exemple une échelle de tolérance 84, par l'intermédiaire de laquelle, le membre d'équipage peut déclarer la contrainte de pilotage que forme ledit paramètre représentatif d'une tolérance à un contexte météorologique courant de vol.

L'échelle de tolérance 84 est graduée, chaque graduation correspondant à des tolérances différentes. L'échelle de tolérance 84 comprend au moins deux graduations d'extrémité correspondant respectivement à une tolérance basse (« Low ») et à une tolérance haute (« High »).

Un membre d'équipage est propre à enclencher un élément graphique 86 associé à la condition givrante de météorologique. Cet élément graphique 86 est par exemple ici intitulé « Avoid rest during icing condition » (traduit par « Eviter le repos pendant les conditions givrantes »).

Lorsque cet élément graphique 86 est actionné, aucune tolérance n'est associée à l'état de condition givrante. En particulier, chaque pilote doit alors être en service en cas de condition givrante.

Une des zones graphiques d'acquisition est une zone 88 de contraintes temporelles de service, par exemple intitulée ici « No rest areas » (traduit par « Zones sans période de repos »).

Comme illustré sur la figure 2, la zone 88 de contraintes temporelles de service comprend par exemple une icône d'ajout 90, par l'actionnement de laquelle, le membre d'équipage peut déclarer la contrainte de pilotage que forme une zone temporelle ou géographique prédéterminée de vol nécessitant un nombre prédéterminé de pilotes, par exemple la totalité, en service.

La zone 88 de contraintes temporelles ou géographique de service comprend, pour chaque zone temporelle déclarée, des icônes de modification et de suppression de la zone temporelle déclarée.

La fenêtre d'acquisition 50 comprend aussi une icône 92 graphique de réinitialisation, par exemple ici intitulée « Reset » (traduit par « Réinitialiser »).

Lorsqu'un membre de l'équipage actionne l'icône 92 graphique de réinitialisation, le module de gestion d'affichage 36 est configuré pour afficher la fenêtre d'acquisition 50 dans une configuration par défaut, ayant par exemple des valeurs par défaut pour chaque zone d'affichage. Lesdites valeurs par défaut sont par exemple stockées dans la mémoire 30.

La fenêtre d'acquisition 50 comprend aussi une icône 94 graphique actionnable de déclenchement de la détermination du scénario de repos, par exemple ici intitulée « Compute » (traduit par « Calculer »).

Une fois qu'un membre de l'équipage actionne l'icône 94 graphique de déclenchement, le module d'acquisition 32 est configuré pour acquérir chacune des entrées de contexte et de contraintes de vol des zones graphiques d'acquisition de la fenêtre 50. Le module d'acquisition 32 est aussi configuré pour acquérir d'autres entrées par interrogation de la mémoire 30 et/ou par l'intermédiaire de capteurs de l'aéronef 12. Le module de détermination 34 est alors configuré pour déterminer le scénario de repos à partir des entrées acquises, comme expliqué plus en détails ci-dessus.

Puis, le module de gestion d'affichage 36 est au moins configuré pour afficher le scénario de repos déterminé sur l'écran 20 du dispositif d'affichage 16, dans une fenêtre de résultats 100, dont un mode de réalisation non limitant est illustré sur la figure 3.

La fenêtre de résultats 100 comprend une pluralité de zones graphiques de résultats.

La fenêtre de résultats 100 comprend par exemple des éléments graphiques actionnables, l'actionnement étant mis en œuvre par l'intermédiaire de l'interface homme-machine 18.

Les éléments graphiques sont actionnables indépendamment les uns des autres.

Dans le mode de réalisation préféré d'un écran tactile, l'actionnement est détecté par le contact desdits éléments graphiques affichés, par un ou plusieurs organe(s) de commande sur la surface de l'écran 20.

Chaque zone graphique de résultats comprend de préférence une forme de cadrant.

Chaque zone graphique de résultats comprend par exemple un titre représentatif du ou des résultats associé(s) à la zone, le titre étant disposé dans un cadrant respectif en regard de la zone. Alternativement, la zone graphique est dépourvue de titre.

La fenêtre de résultats 100 n'est pas limitée au mode de réalisation qui est illustré sur la figure 3 et décrit ci-dessous. Elle peut comprendre en particulier toute autre zone graphique.

Une des zones graphiques de résultats est une zone 102 d'alerte de concordance des entrées acquises, par exemple ici intitulée « Compliance » (traduit par « Compatibilité »).

Dans la zone d'alerte 102, le module de gestion d'affichage 36 est configuré pour générer une représentation graphique 104 de l'alerte qui est générée par le module de détermination 34 dans le cas où une condition de compatibilité entre entrées acquises n'est pas vérifiée. Pour ce faire, le module de gestion d'affichage 36 est configuré pour recevoir le signal représentatif de la vérification de chaque condition de compatibilité, qui est généré par le module de détermination 34.

L'alerte affichée 104 est aussi par exemple représentative, le cas échéant, de chaque modification des entrées faite par le module de détermination 34.

L'alerte affichée 104 prend par exemple la forme d'un message textuel et/ou d'un élément graphique de couleur.

Dans la zone d'alerte 102, le module de gestion d'affichage 36 est aussi avantageusement configuré pour afficher un message textuel et/ou un élément graphique de couleur, dans le cas où chaque condition de compatibilité entre entrées acquises est vérifiée.

Dans l'exemple illustré sur la figure 3, chaque condition de compatibilité est vérifiée, la zone 102 affichant un élément graphique de couleur vert et le message textuel « Rest scenario compatible with defined constraints » (traduit par « Scénario de repos compatible avec contraintes définies »).

Une des zones graphiques de résultats est une zone 106 de scénario de repos déterminé, par exemple ici intitulée « Recommended Rest Scenario » (traduit par « Scénario de repos recommandé »).

Dans la zone 106 de scénario, le module de gestion d'affichage 36 est configuré pour afficher au moins une représentation graphique du scénario déterminé comprenant la chronologie compatible ayant le meilleur classement selon ladite relation d'ordre.

Plusieurs modes de réalisation de la représentation graphique 108A, 108B du scénario déterminé vont être décrits.

Dans un premier mode de réalisation 108A illustré sur la figure 3, la représentation graphique 108A du scénario déterminé comprend, pour chaque pilote de l'équipage, l'instant de début et l'instant de fin de chaque plage de repos.

Le premier mode de représentation graphique 108A comprend aussi, pour chaque pilote, un identifiant graphique représentatif du pilote auquel est associé chaque plage de repos.

Dans un deuxième mode de réalisation 108B, aussi illustré sur la figure 3, la représentation graphique 108B du scénario déterminé comprend par exemple, pour chaque pilote de l'équipage, une ligne de temps 110 sur laquelle est affichée chaque plage de service et chaque plage de repos du scénario déterminé.

Le deuxième mode de représentation graphique 108B comprend au moins une échelle temporelle 112 disposée en regard des lignes de temps 110.

L'échelle temporelle 112 est graduée, chaque graduation correspondant à un instant du vol. Dans l'exemple illustré, chaque graduation est séparée d'une heure.

Les plages sont placées sur chaque ligne de temps 110 en fonction de leurs instants de début et de fin respectifs, par rapport à l'échelle temporelle 112.

Le deuxième mode de représentation graphique 108B comprend aussi, pour chaque ligne de temps 110, un identifiant graphique représentatif du pilote auquel est associée ladite ligne de temps 110. L'identifiant est disposé en regard de la ligne de temps 110 associée.

Avantageusement, la durée de sommeil autorisé et la durée de transition de poste sont affichées différemment.

Dans l'exemple de la figure 3, la durée de sommeil autorisé est illustrée par une portion de la ligne de temps 100 avec une haute densité de hachures et la durée de transition de poste est illustrée par une portion de la ligne de temps 100 avec une plus faible densité de hachures.

Avantageusement, le deuxième mode de représentation graphique 108B du scénario de repos déterminé est représentatif de l'évolution du niveau de fatigue de chaque pilote au cours de chaque plage de service du pilote.

Le deuxième mode de représentation graphique 108B comprend alors par exemple, pour chaque plage de service affichée, un dégradé de couleur représentatif de ladite évolution au cours de la plage de service.

Dans l'exemple de la figure 3, cette évolution a été illustrée par un changement de densité de pointillés.

Le deuxième mode de représentation graphique 108B comprend aussi par exemple des éléments graphiques représentatifs d'au moins une des entrées de contexte et de contrainte de vol acquises, disposés le long d'au moins une des lignes de temps 110.

Avantageusement, ces éléments graphiques sont représentatifs d'au moins une des informations relatives au vol acquises. Ainsi, dans l'exemple illustré sur la figure 3, la représentation graphique 108B du scénario déterminé comprend un élément graphique 114 représentatif du sommet de montée et un élément graphique 116 représentatif du début de descente.

La représentation graphique 108B du scénario déterminé comprend aussi par exemple un élément graphique 118 représentatif d'un des événements destinés à se produire lors du vol, représentatif d'une des zones temporelles prédéterminées de vol nécessitant un nombre prédéterminé de pilotes en service et/ou représentatif d'une des zones temporelles de vol nécessitant un pilote prédéterminé en service parmi les pilotes de l'équipage.

La zone 106 de scénario comprend de préférence une icône d'aide 120 actionnable, par exemple intitulée ici « ? Guideline » (traduit par « ? Mode d'emploi »). Lorsque l'icône d'aide est actionnée, le module de gestion d'affichage 36 est configuré pour afficher des informations d'aide à la lecture de l'affichage, les informations comprenant par exemple des descriptifs de chaque élément graphique affiché, et commandes disponibles avec description de leurs effets.

Dans un troisième mode de réalisation, non illustré, la représentation graphique du scénario de repos déterminé comprend une carte géographique survolée au cours du vol et un trajet de vol de l'aéronef 12 au-dessus de la carte, chaque plage de repos de la chronologie du scénario déterminé étant affichée en superposition sur le trajet de vol.

Le trajet s'étend par exemple entre le décollage et l'atterrissage, ou entre un instant courant pendant le vol et l'atterrissage.

Les plages sont placées sur le trajet de vol en fonction de leurs instants de début et de fin respectifs.

Chaque plage présente par exemple alors un identifiant graphique représentatif du pilote auquel est associé ladite plage.

De plus, avantageusement, le troisième mode de représentation graphique est aussi représentatif de l'évolution du niveau de fatigue de chaque pilote au cours de chaque plage de service du pilote.

De préférence, le module de gestion d'affichage 36 est configuré pour passer d'un mode d'affichage comprenant au moins une représentation graphique du scénario de repos dans un des modes de réalisation décrits ci-dessus, à un autre mode d'affichage comprenant au moins une représentation graphique du scénario dans un autre des modes de réalisation.

Dans l'exemple illustré sur la figure 3, le module de gestion d'affichage 36 est configuré pour passer d'un mode d'affichage comprenant des représentations graphiques du scénario de repos selon les premier et deuxième modes de réalisation à un autre mode d'affichage comprenant une représentation graphique du scénario selon le troisième mode de réalisation.

Pour cela, la zone 106 de scénario de repos comprend de préférence une icône 122 d'affichage de carte, par exemple intitulée ici « Display on map » (traduit par « Afficher sur la carte »). Lorsque l'icône d'affichage 122 est actionnée, le module de gestion d'affichage 36 est configuré pour afficher la représentation graphique du scénario déterminé suivant le troisième mode de réalisation décrit ci-dessus.

En complément, il est possible à l'équipage de modifier manuellement le scénario déterminé par le module de détermination 34.

Le module de gestion d'affichage 36 est ainsi configuré pour détecter une action de modification du scénario de repos affiché, mise en œuvre par un opérateur par l'intermédiaire de l'interface homme-machine 18, et pour modifier l'affichage en conséquence.

L'action de modification du scénario de repos affiché comprend par exemple un ajout et/ou une suppression d'une plage de repos dans le scénario de repos déterminé.

Pour ce faire, dans l'exemple illustré de la figure 3, la zone 106 de scénario de repos comprend une icône d'ajout 124, ici intitulée « Add new » (traduit par « ajouter »). Lorsque l'icône d'ajout 124 est actionnée, le module de gestion d'affichage 36 est configuré pour afficher une fenêtre permettant à l'opérateur de définir manuellement une nouvelle plage de repos qui sera ajoutée au scénario de repos déterminé.

De plus, dans l'exemple illustré de la figure 3, la zone 106 de scénario de repos comprend aussi icône de suppression 126. Lorsque l'icône de suppression 126 est actionnée, le module de gestion d'affichage 36 est configuré pour sélectionné et supprimer une des plages de repos du scénario déterminé.

L'action de modification du scénario de repos affiché comprend par exemple une modification d'une position temporelle d'au moins une des plages de repos du scénario.

Pour ce faire, l'action de modification comprend par exemple un déplacement d'un organe par un opérateur sur l'écran tactile dans une direction sensiblement parallèle à l'échelle temporelle 112, l'organe contactant l'écran 20 en regard de la plage de repos à modifier.

Ainsi, la plage de repos à modifier est déplacée temporellement, sur la ligne de temps 110 associée. Elle est en particulier déplacée vers l'amont ou vers l'aval du vol, en fonction du sens du déplacement de l'organe.

A l'issue de ce déplacement temporel, la plage de repos modifiée comprend un instant modifié de début et à un instant modifié de fin.

Après modification, en particulier après l'ajout, la suppression et/ou le déplacement temporel, le module de détermination 34 est alors propre à simuler et déterminer un niveau de fatigue de chaque pilote à partir du modèle biomathématique de fatigue dans la chronologie modifiée du scénario de repos modifié.

Puis, le module de gestion d'affichage 36 est configuré pour afficher le scénario de repos modifié, selon une représentation graphique spécifique. Cette représentation graphique spécifique est par exemple selon un des modes de réalisation décrits ci-dessus.

La fenêtre de résultats 100 comprend aussi par exemple une icône 128 graphique d'impression actionnable, par exemple ici intitulée « Print » (traduit par « Imprimer »). Lorsqu'un membre de l'équipage actionne l'icône 128 d'impression, le module de gestion d'affichage 36 est configuré pour déclencher une impression papier représentative de la fenêtre de résultats 100. Cette impression est par exemple mise en œuvre par l'imprimante de l'aéronef 12.

En outre, la fenêtre de résultats 100 comprend aussi par exemple une icône 130 graphique de retour actionnable, par exemple ici intitulée « Modify mission » (traduit par « Modifier la mission »). Lorsqu'un membre de l'équipage actionne l'icône 130 graphique de retour, le module de gestion d'affichage 36 est configuré pour afficher la fenêtre d'acquisition 50 la place de la fenêtre de résultats 100.

Dans un exemple d'utilisation, après avoir obtenu une première fenêtre de résultats 100 avant vol, l'icône 130 graphique de retour peut par exemple être actionnée pendant le vol pour relancer la détermination du scénario en fonction de l'évolution de la mission, avantageusement si la mission diffère de sa prévision avant vol.

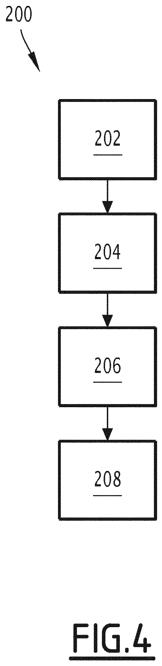

Une méthode 200 de détermination d'un scénario de repos en vol d'un équipage d'aéronef 12 va maintenant être décrite en référence à la figure 4. De préférence, la méthode 200 utilise le système de détermination 10 tel que décrit ci-dessus.

La méthode 200 sera illustrée par plusieurs exemples.

La méthode 200 comprend une étape 202 d'acquisition des entrées de contexte et de contraintes de vol, telles que celles décrites ci-dessus.

L'étape 202 d'acquisition est par exemple mise en œuvre par le module d'acquisition 32 décrit ci-dessus.

Dans un premier exemple illustratif, la méthode 200 est mise en œuvre avant l'instant de sommet de montée TOC, en particulier avant le vol.

Dans le premier exemple illustratif, les entrées acquises sont notamment celles illustrées sur la figure 2. Les entrées comprennent :
- un instant calculé de sommet de montée TOC lors du vol, ayant lieu à 10h00,
- un instant calculé de début de descente TOD lors du vol, ayant lieu à 16h00,
- une durée de croisière disponible, qui est donc ici égale à 6h00,
- un nombre de pilotes de l'équipage égale à 2, les pilotes étant désignés par la suite par P1 et P2,
- un état de fatigue avant-vol déclaré acceptable pour le pilote P1, un état de fatigue avant-vol déclaré mineur pour le pilote P2,
- une durée de plage de repos égale à 2h20, dont 2h de durée de sommeil autorisé et 0h20 de durée de transition de poste,
- un nombre de plage(s) de repos à prévoir par pilote égal à 1, ce nombre étant le même pour chaque pilote, le nombre de plage(s) de repos nécessaire(s) à prévoir pour l'équipage étant donc égal à 2,
- une tolérance intermédiaire à un contexte météorologique courant,
- une zone temporelle de vol nécessitant les deux pilotes en service entre 12h30 et 13h00,
- le nombre minimum requis de pilote(s) en service aux commandes de l'aéronef 12 à chaque instant de la croisière est égal à 1.

Après l'étape 202 d'acquisition, la méthode 200 comprend une étape 204 de vérification d'au moins une condition de compatibilité entre au moins deux des entrées acquises.

Dans le premier exemple, cette étape 204 vérifie en particulier que la durée de croisière disponible est suffisamment longue pour inclure chaque plage de repos.

Cette condition est ici vérifiée dans la mesure où la somme des durées de chaque plage de repos (4h40) est inférieure à la durée de croisière disponible (6h00).

Aucune alerte n'est donc générée dans ce premier exemple.

La méthode 200 comprend alors une étape 206 de détermination du scénario de repos de l'équipage pour le vol à partir desdites entrées acquises, ladite étape 206 étant par exemple mise en œuvre par le module de détermination 34 décrit ci-dessus.

Comme expliqué plus en détails ci-dessous, cette détermination est faite à partir d'au moins une simulation par le modèle biomathématique de fatigue ayant pour variables au moins une desdites entrées acquises.

L'étape 206 de détermination du scénario comprend la détermination d'au moins une chronologie de plage(s) de repos compatible avec chaque contrainte de pilotage acquise.

Pour cela, la durée de croisière disponible acquise est discrétisée en une pluralité d'instants discrétisés avec un pas de discrétisation prédéterminé.

Par exemple, le pas de discrétisation est calculé pour des paramètres de contraintes de calcul comprenant une durée maximale de calcul de 5s, une durée de calcul par chronologie estimée à 0,1s, un nombre de plage(s) de repos à prévoir égal à 2, une durée de chaque plage de repos égale à 2h20 et la durée de croisière disponible égale à 6h00.

Le nombre maximal de chronologies à calculer permettant de respecter la contrainte de durée maximale de calcul est donc de 50 (5s divisé par 0,1s).

Le nombre maximum de positions temporelles par plage permettant de respecter la contrainte de durée maximale de calcul est donc environ 7 (racine carrée de 50).

Pour une croisière de 6h et une durée de repos de 2h20, cela donne un pas de discrétisation de (6h00 - 2h20)/(7-1) = ~0,6h, soit environ 36 minutes.

Par souci de simplification dans cet exemple, le pas de discrétisation est arrondi à l'heure supérieure et sera considéré ici à 1h00.

L'étape 206 de détermination comprend par la suite le listage de toutes les chronologies de plages de repos dans lesquelles chaque plage de repos a un instant de début correspondant à un des instants discrétisés. Dans chaque chronologie listée, les plages de la chronologie listée ont toutes un instant de fin antérieur à la fin de la phase de croisière, en particulier antérieur à l'instant calculé de début de descente.

Avec un pas de discrétisation est égal à 1h00, les chronologies listées dans le premier exemple comprennent donc uniquement des plages ayant pour instant de début : 10h00, 11h00, 12h00 et 13h00.

En particulier, les chronologies listées ici sont réunies dans le tableau 1 ci-dessous.

**[Table 1] Chronologies listées dans l'exemple illustratif**

| P1 | P2 |
|---|---|
| 10h-12h20 | 10h-12h20 |
| 10h-12h20 | 11h-13h20 |
| 10h-12h20 | 12h-14h20 |
| 10h-12h20 | 13h-15h20 |
| 11h-13h20 | 10h-12h20 |
| 11h-13h20 | 11h-13h20 |
| 11h-13h20 | 12h-14h20 |
| 11h-13h20 | 13h-15h20 |
| 12h-14h20 | 10h-12h20 |
| 12h-14h20 | 11h-13h20 |
| 12h-14h20 | 12h-14h20 |
| 12h-14h20 | 13h-15h20 |
| 13h-15h20 | 10h-12h20 |
| 13h-15h20 | 11h-13h20 |
| 13h-15h20 | 12h-14h20 |
| 13h-15h20 | 13h-15h20 |

L'étape 206 de détermination du scénario comprend alors la vérification, pour chaque chronologie listée, si la chronologie listée est compatible avec chaque contrainte de pilotage acquise.

Dans l'exemple illustré, les contraintes de pilotage considérées sont notamment la zone temporelle de vol nécessitant les deux pilotes en service entre 12h30 et 13h00, et le nombre minimum égal à 1 de pilote(s) requis en service aux commandes de l'aéronef 12 à chaque instant de la croisière. En d'autres termes, les plages de repos ne peuvent pas se superposer.

Ainsi, les chronologies listées compatibles ici sont réunies dans le tableau 2 ci-dessous.

**[Table 2] Chronologies listées compatibles dans l'exemple illustratif**

| | P1 | P2 |
|---|---|---|
| Chronologie 1 | 10h-12h20 | 13h-15h20 |
| Chronologie 2 | 13h-15h20 | 10h-12h20 |

L'étape 206 de détermination comprend alors la simulation et la détermination, pour chaque chronologie compatible, d'au moins un niveau de fatigue de chaque pilote, par le modèle biomathématique.

Avantageusement, l'étape 206 de détermination comprend la simulation et la détermination, par le modèle biomathématique et pour chaque chronologie compatible, d'un niveau de fatigue maximal de chaque pilote en service pendant chaque plage de repos de chaque autre pilote et/ou un niveau de fatigue à un instant calculé de début de descente (TOD) de l'aéronef 12 d'au moins chaque pilote prévu en service audit instant.

Le modèle biomathématique de fatigue quantifie chaque niveau de fatigue sur une échelle de fatigue prédéterminée. Le modèle biomathématique est ici le modèle SWP.

Dans l'exemple illustratif, les niveaux de fatigue simulés pour chaque chronologie compatible sont réunis dans le tableau 3 ci-dessous. Par « Fatigue max P1 pendant repos P2 », on entend le niveau de fatigue maximal du pilote P1 en service pendant la plage de repos du pilote P2. De plus, par « Fatigue P1 au TOD », on entend le niveau de fatigue à l'instant calculé de début de descente (TOD) du pilote P1.

**[Table 3] Etats de fatigue simulés et déterminés pour chaque chronologie compatible dans l'exemple illustratif**

| | P1 | P2 | Fatigue max P1 pendant repos P2 | Fatigue max P2 pendant repos P1 | Fatigue P1 au TOD | Fatigue P2 au TOD |
|---|---|---|---|---|---|---|
| chronologie 1 | 10h-12h20 | 13h-15h20 | 5,8 | 4,1 | 4,8 | 4,5 |
| chronologie 2 | 13h-15h20 | 10h-12h20 | 5 | 5,5 | 4 | 5,2 |

Après simulation, l'étape 206 de détermination comprend le classement de chaque chronologie compatible selon une relation d'ordre établie à partir des niveaux de fatigue simulés.

La relation d'ordre fait par exemple ici intervenir, dans chaque chronologie compatible, la moyenne desdits niveaux de fatigue maximaux simulés de chaque pilote en service et la moyenne desdits niveaux de fatigue au TOD.

La relation d'ordre comprend alors une moyenne pondérée des rangs obtenus.

Dans l'exemple illustratif, la pondération du rang associé pour les niveaux de fatigue au TOD est deux fois plus importante que celle du rang associé pour les niveaux de fatigue maximaux simulés de chaque pilote en service.

Dans l'exemple illustratif, les rangs obtenus sont réunis dans le tableau 4 ci-dessous.

**[Table 4] Rangs obtenus dans l'exemple illustratif**

| | Fatigue max P1 pendant repos P2 | Fatigue max P2 pendant repos P1 | Moyenne | Rang | Fatigue P1 au TOD | Fatigue P2 au TOD | Moyenne | Rang | Rang global (pondération des rangs) |
|---|---|---|---|---|---|---|---|---|---|
| chronologie 1 | 5,8 | 4,1 | 4,95 | 1 | 4,8 | 4,5 | 4,65 | 2 | 1,67 |
| chronologie 2 | 5 | 5,5 | 5,25 | 2 | 4 | 5,2 | 4,6 | 1 | 1,33 |

A l'issue de l'étape 206 de détermination, le scénario de repos déterminé comprend la chronologie compatible ayant le meilleur classement selon la relation d'ordre.

Dans l'exemple illustratif, le meilleur classement est la chronologie ayant le « rang global » le plus bas pour minimiser la fatigue.

Le scénario déterminé, dans cet exemple, comprend donc la chronologie numérotée 2, et comprend donc une plage de repos du pilote P2 entre 10h-12h20 et une plage de repos du pilote P1 entre 13h-15h20.

La méthode 200 comprend alors une étape 208 d'affichage du scénario de repos déterminé sur l'écran 20 du dispositif d'affichage 16.

Cette étape 208 d'affichage est mise en œuvre par exemple par le module de gestion d'affichage 36 décrit ci-dessus.

La figure 3 illustre notamment une représentation graphique du scénario déterminé dans l'exemple illustratif.

Un deuxième exemple illustratif de la méthode 200 va maintenant être décrit.

Dans le deuxième exemple illustratif, la méthode 200 est mise en œuvre pendant la croisière à un instant courant. Dans le deuxième exemple, le pilote P2 a ici réalisée une plage de repos avant cet instant courant.

Les entrées acquises pour le deuxième exemple diffèrent donc de celles du premier en ce que la durée de croisière disponible correspond à l'écart entre l'instant courant du vol et l'instant calculé de début de descente TOD.

Les entrées acquises pour le deuxième exemple comprennent donc :
- l'instant courant ayant lieu à 14h00, à savoir avant l'instant calculé de début de descente TOD ayant lieu à 16h00,
- une durée de croisière disponible donc de 2h00,
- un nombre de plage(s) de repos à prévoir pour le pilote P1 égal à 1 et un nombre de plage(s) de repos à prévoir pour le pilote P2 égal à 0, le nombre de plage(s) de repos nécessaire(s) à prévoir pour l'équipage étant donc égal à 1.

Les autres entrées acquises pour le deuxième exemple sont similaires à celles du premier exemple illustratif.

Après l'étape 202 d'acquisition, la méthode 200 comprend l'étape 204 de vérification de la condition de compatibilité selon laquelle la durée de croisière disponible est suffisamment longue pour inclure chaque plage de repos.

Dans le deuxième exemple, cette condition n'est pas vérifiée, dans la mesure où la durée de la seule plage de repos à prévoir (2h20) est supérieure à la durée de croisière disponible (2h00).

La méthode 200 comprend donc la génération d'une alerte à destination de l'équipage et la modification de la durée de plage de repos acquise.

Dans ce cas, la durée de sommeil autorisé de la plage de repos est réduite, en conservant la durée de transition de poste prédéterminée.

Dans le deuxième exemple, la durée de plage de repos est modifiée pour être réduite à 2h00, en réduisant la durée de sommeil autorisé à 1h40 et en conservant la durée de transition de poste à 0h20.

L'étape 206 de détermination du scénario comprend la détermination d'au moins une chronologie de plage(s) de repos compatible avec chaque contrainte de pilotage acquise, mais dans le cas présent, il n'existe qu'une seule chronologie possible, à savoir une chronologie comprenant une plage de repos pour le pilote P1 entre 14h00 et 16h00.

L'étape 206 de détermination comprend alors la simulation et la détermination, pour ladite chronologie compatible, de chaque niveau de fatigue de chaque pilote, par le modèle biomathématique.

Après simulation, l'étape 206 de détermination comprend ledit classement qui est ici direct, dans la mesure où il n'y a qu'une chronologie compatible.

La méthode 200 comprend alors l'étape 208 d'affichage du scénario de repos déterminé sur l'écran 20 du dispositif d'affichage 16, et l'affichage d'une représentation graphique de l'alerte générée. L'alerte affichée est aussi représentative de la modification de la durée de plage de repos.

Des variantes et compléments du système de détermination ci-dessus vont maintenant être décrits.

Alternativement, l'équipage comprend au moins trois pilotes, par exemple exactement trois pilotes. Encore en variante, l'équipage comprend au moins quatre pilotes, par exemple exactement quatre.

Dans une variante, l'écran 20 du dispositif d'affichage 16 n'est pas tactile.

En variante, l'interface homme machine 18 comprend une souris et un clavier. L'interface 18 est alors notamment configurée pour détecter la position, sur une surface de l'écran 20, d'un pointeur graphique contrôlé par la souris.

Dans une autre variante, le système de détermination 10 n'est pas compris dans l'aéronef 12.

Dans une variante avantageuse, non illustrée, le système embarqué de l'aéronef 12 comprend un dispositif de réveil d'un pilote au repos et module de réveil est configuré pour actionner le dispositif de réveil.

Le dispositif de réveil étant propre à être actionné pour réveiller un des pilotes lorsqu'il est dans sa plage de repos.

Le système de détermination 10 est ainsi connecté au dispositif de réveil

Le module de réveil est configuré pour actionner le dispositif de réveil d'un pilote au repos en fonction du scénario de repos déterminé.

Le module de réveil est en particulier configuré pour actionner le dispositif de réveil en fonction d'un instant de fin de la durée de sommeil autorisé de chaque plage de repos.

Dans une variante non illustrée, le module de gestion d'affichage 36 est configuré pour afficher en outre au moins un scénario de repos alternatif. Le scénario de repos alternatif comprend alors la chronologie compatible ayant le deuxième meilleur classement, selon la relation d'ordre.

De manière plus générale, le module de gestion d'affichage 36 est avantageusement configuré pour afficher en outre un nombre prédéterminé de scénarios de repos alternatifs, comprenant respectivement les chronologies compatibles ayant les meilleurs classements, selon la relation d'ordre.

Dans une autre variante, après avoir déterminé la chronologie compatible ayant le meilleur classement, le module de détermination 34 est configuré pour affiner la détermination du scénario, par exemple en mettant en œuvre une nouvelle fois chaque étape à partir de l'étape de discrétisation de la durée de croisière disponible.

En effet, le fait d'avoir déterminé la chronologie de meilleur classement permet de réduire le pas de discrétisation, dans la mesure où il n'est plus nécessaire de discrétiser l'ensemble de la durée de croisière disponible et il est possible de ne discrétiser que des fenêtres temporelles autour de chaque plage de repos.

Plus précisément, le module de détermination 34 est configuré pour déterminer, pour chaque plage de ladite chronologie compatible ayant le meilleur classement, une fenêtre temporelle incluant la plage.

Le module de détermination 34 est alors configuré pour discrétiser, avec un pas de discrétisation réduit, chaque fenêtre temporelle en une pluralité d'instants discrétisés réduits.

Le pas de discrétisation réduit est inférieur au pas de discrétisation prédéterminé.

Le module de détermination 34 est ensuite configuré pour lister, après discrétisation réduite, toutes les nouvelles chronologies de plages de repos dans lesquelles chaque plage de repos a un instant de début correspondant à un des instants discrétisés réduits. Chaque plage de repos est incluse dans une desdites fenêtres temporelles.

Le module de détermination 34 est configuré pour vérifier, pour chaque nouvelle chronologie listée, si la nouvelle chronologie listée est compatible avec chaque contrainte de pilotage.

De plus, le module de détermination 34 est configuré pour simuler et déterminer, pour chaque nouvelle chronologie compatible, au moins un nouveau niveau de fatigue de chaque pilote, par le modèle biomathématique.

Par la suite, le module de détermination 34 est configuré pour classer chaque chronologie nouvelle compatible selon la relation d'ordre établie à partir des nouveaux niveaux de fatigue simulés.

Le scénario de repos déterminé par le module de détermination 34 est alors le scénario comprenant la chronologie nouvelle compatible ayant le meilleur classement selon ladite relation d'ordre.

Grâce aux caractéristiques précédemment décrites, le système de détermination 10 permet d'améliorer la sécurité de l'aéronef 12 en minimisant la fatigue de l'équipage pendant le vol mettant en œuvre des périodes de repos.

En particulier, le système propose le scénario de repos le plus opportun et efficace possible, tout en respectant les besoins physiologiques et les diverses contraintes du vol telles que les exigences règlementaires.

Le système de détermination 10 est robuste au nombre de pilotes et à l'ajout de tout autre nouveau critère de minimisation.

Le système de détermination 10 permet aussi aux membres de l'équipage de modifier le scénario proposé et de visualiser les effets de chaque modification sur le niveau de fatigue de l'équipage.

## Revendications

1. Système (10) de détermination d'un scénario de repos en vol d'un équipage d'aéronef (12), l'équipage comprenant au moins deux pilotes propres à piloter l'aéronef (12) pendant un vol de l'aéronef (12), le système (10) comprenant :
- un dispositif d'affichage (16) comprenant un écran (20),
- un module (32) d'acquisition d'entrées de contexte et de contraintes de vol,
- un module (34) de détermination d'au moins un scénario de repos de l'équipage pour le vol à partir desdites entrées acquises, le module de détermination (34) étant propre à déterminer ledit scénario de repos à partir d'au moins une simulation par un modèle biomathématique de fatigue ayant pour variables au moins une desdites entrées acquises,
- un module (36) de gestion d'affichage, configuré pour afficher le scénario de repos déterminé sur l'écran (20) du dispositif d'affichage (16) ;
**caractérisé en ce que** le scénario de repos est déterminé pour une phase croisière du vol et comprend une chronologie de plage(s) de repos, chaque plage étant attribuée à un des pilotes, et les entrées de contexte et de contraintes de vol comprennent au moins une contrainte de pilotage par l'équipage de l'aéronef (12),
le module de détermination (34) étant configuré pour déterminer au moins une chronologie de plage(s) de repos compatible avec chaque contrainte de pilotage,
le module de détermination (34) étant configuré pour simuler et déterminer, pour chaque chronologie compatible, au moins un niveau de fatigue de chaque pilote, par le modèle biomathématique,
le module de détermination (34) étant configuré pour classer chaque chronologie compatible selon une relation d'ordre établie à partir des niveaux de fatigue simulés, le scénario de repos déterminé comprenant la chronologie compatible ayant le meilleur classement selon ladite relation d'ordre.

2. Système (10) selon la revendication 1, dans lequel la ou une des contraintes de pilotage est un nombre minimum requis de pilote(s) en service à chaque instant de la croisière ;
et/ou la ou une des contraintes de pilotage est une zone temporelle ou géographique prédéterminée de vol nécessitant un nombre prédéterminé de pilotes en service, le nombre prédéterminé étant par exemple la totalité des pilotes de l'équipage ;
et/ou la ou une des contraintes de pilotage est une zone temporelle ou géographique de vol nécessitant un pilote prédéterminé en service parmi les pilotes de l'équipage ;
et/ou la ou une des contraintes de pilotage est un paramètre représentatif d'une tolérance à un contexte météorologique courant de vol.

3. Système (10) selon l'une quelconque des revendications 1 ou 2, dans lequel les entrées de contexte et de contraintes de vol comprennent, pour chaque pilote, au moins un paramètre de fatigue avant-vol représentatif d'un état de fatigue avant-vol déclaré par le pilote, le modèle biomathématique ayant comme variable ledit paramètre de fatigue avant-vol.

4. Système (10) selon l'une quelconque des revendications 1 à 3, dans lequel le module de détermination (34) est configuré pour simuler et déterminer, par le modèle biomathématique et pour chaque chronologie compatible, un niveau de fatigue maximal de chaque pilote en service pendant chaque plage de repos de chaque autre pilote et/ou un niveau de fatigue à un instant calculé de début de descente de l'aéronef (12) d'au moins chaque pilote prévu en service audit instant, la relation d'ordre étant établie sur au moins un desdits niveaux de fatigue simulés et déterminés.

5. Système (10) selon l'une quelconque des revendications 1 à 4, dans lequel les entrées de contexte et de contraintes de vol comprennent une durée de croisière disponible, un nombre de plage(s) de repos nécessaire(s) à prévoir pour l'équipage et une durée de plage de repos,
le module de détermination (34) étant configuré pour discrétiser la durée de croisière disponible en une pluralité d'instants discrétisés, la discrétisation étant faite avec un pas de discrétisation,
le module de détermination (34) étant configuré pour déterminer chaque chronologie compatible de sorte que chaque plage de repos de la chronologie compatible ait un instant de début correspondant à un des instants discrétisés.

6. Système (10) selon la revendication 5, dans lequel le module de détermination (34) est configuré pour lister, après discrétisation, toutes les chronologies de plages de repos dans lesquelles chaque plage de repos a un instant de début correspondant à un des instants discrétisés, et pour vérifier, pour chaque chronologie listée, si la chronologie listée est compatible avec chaque contrainte de pilotage.

7. Système (10) selon la revendication 6, dans lequel, après avoir déterminé la chronologie compatible ayant le meilleur classement, le module de détermination (34) est configuré pour déterminer, pour chaque plage de ladite chronologie, une fenêtre temporelle incluant la plage,
le module de détermination (34) étant configuré pour discrétiser, avec un pas de discrétisation réduit, chaque fenêtre temporelle en une pluralité d'instants discrétisés réduits, le pas de discrétisation réduit étant inférieur au pas de discrétisation prédéterminé,
le module de détermination (34) étant configuré pour lister, après discrétisation réduite, toutes les nouvelles chronologies de plages de repos dans lesquelles chaque plage de repos a un instant de début correspondant à un des instants discrétisés réduits, et pour vérifier, pour chaque nouvelle chronologie listée, si la nouvelle chronologie listée est compatible avec chaque contrainte de pilotage,
le module de détermination (34) étant configuré pour simuler et déterminer, pour chaque nouvelle chronologie compatible, au moins un nouveau niveau de fatigue de chaque pilote, par le modèle biomathématique,
le module de détermination (34) étant configuré pour classer chaque chronologie nouvelle compatible selon la relation d'ordre établie à partir des nouveaux niveaux de fatigue simulés, le scénario de repos déterminé comprenant la chronologie nouvelle compatible ayant le meilleur classement selon ladite relation d'ordre.

8. Système (10) selon la revendication 1 à 7, dans lequel, avant de déterminer chaque chronologie de plage(s) de repos compatible avec chaque contrainte de pilotage acquise, le module de détermination (34) est configuré pour vérifier au moins une condition de compatibilité entre au moins deux des entrées acquises, et configuré pour modifier une des entrées acquises si la condition de compatibilité entre entrées n'est pas vérifiée, et/ou pour générer une alerte à destination de l'équipage si la condition de compatibilité entre entrées n'est pas vérifiée, le module de gestion d'affichage (36) étant configuré pour générer une représentation graphique de l'alerte.

9. Système (10) selon la revendication 8, dans lequel les entrées de contexte et de contraintes de vol comprennent une durée de croisière disponible, un nombre de plage(s) de repos nécessaire(s) à prévoir pour l'équipage, et une durée de plage de repos ; le module de détermination (34) étant configuré pour vérifier une condition de compatibilité entre la durée de croisière disponible et les plages de repos à prévoir, la condition de compatibilité étant que la durée de croisière disponible est suffisamment longue pour inclure chaque plage de repos,
le module de détermination (34) étant de préférence configuré pour générer une alerte à destination de l'équipage, si la durée de croisière disponible est trop courte pour inclure chaque plage de repos, et/ou configuré pour réduire la durée d'au moins une des plages de repos à prévoir, si la durée de croisière disponible est trop courte pour inclure chaque plage de repos.

10. Système (10) selon l'une quelconque des revendications 1 à 9, dans lequel chaque plage de repos comprend une durée de sommeil autorisé et une durée de transition de poste.

11. Système (10) selon l'une quelconque des revendications 1 à 10, dans lequel le scénario de repos déterminé comprend aussi, pour chaque pilote, des plages de service, le module de détermination (34) étant configuré pour simuler et déterminer, par le modèle biomathématique, une évolution d'un niveau de fatigue de chaque pilote au cours de chaque plage de service du pilote, le module de gestion d'affichage (36) étant configuré pour afficher, sur l'écran (20) du dispositif d'affichage (16), une représentation graphique spécifique du scénario de repos déterminé représentative de chaque évolution.

12. Système (10) selon l'une quelconque des revendications précédentes, dans lequel le système de détermination (10) comprend une interface homme machine (18) et le module de gestion d'affichage (36) est configuré pour afficher, sur l'écran (20) du dispositif d'affichage (16), une fenêtre (50) comprenant une pluralité de zones graphiques d'acquisition d'entrées, chaque zone graphique d'acquisition d'entrées étant associée à au moins une des entrées de contexte et de contraintes de vol, le module d'acquisition (32) étant configuré pour acquérir au moins une partie des entrées de contexte et de contraintes de vol par l'actionnement d'éléments graphiques affichés dans chaque zone d'acquisition, l'actionnement étant mis en œuvre par l'intermédiaire de l'interface homme-machine (18).

13. Système (10) selon l'une quelconque des revendications précédentes, dans lequel le système de détermination (10) comprend une interface homme machine (18) et ledit module de gestion d'affichage (36) est configuré pour détecter une action de modification du scénario de repos affiché, mise en œuvre par un opérateur par l'intermédiaire de l'interface homme-machine (18),
le module de détermination (34) étant propre à simuler et déterminer un niveau de fatigue de chaque pilote à partir du modèle biomathématique de fatigue dans le scénario de repos modifié, le module de gestion d'affichage (36) étant configuré pour afficher le scénario de repos modifié, selon une représentation graphique spécifique.

14. Méthode (200) de détermination d'un scénario de repos en vol d'un équipage d'aéronef (12), l'équipage comprenant au moins deux pilotes propres à piloter l'aéronef (12) pendant un vol de l'aéronef (12), la méthode (200) comprenant les étapes suivantes :
- acquisition (202) d'entrées de contexte et de contraintes de vol,
- détermination (206) d'au moins un scénario de repos de l'équipage pour le vol à partir desdites entrées acquises, ledit scénario de repos étant déterminé à partir d'au moins une simulation par un modèle biomathématique de fatigue ayant pour variables au moins une desdites entrées acquises, et
- affichage du scénario de repos déterminé sur un écran (20) d'un dispositif d'affichage (16) ;
**caractérisée en ce que** le scénario de repos est déterminé pour une phase croisière du vol et comprend une chronologie de plage(s) de repos, chaque plage étant attribuée à un des pilotes, et les entrées de contexte et de contraintes de vol comprennent au moins une contrainte de pilotage par l'équipage de l'aéronef (12),
la détermination (206) comprenant :
* la détermination d'au moins une chronologie de plage(s) de repos compatible avec chaque contrainte de pilotage,
* la simulation et la détermination, pour chaque chronologie compatible, d'au moins un niveau de fatigue de chaque pilote, par le modèle biomathématique,
* le classement de chaque chronologie compatible selon une relation d'ordre établie à partir des niveaux de fatigue simulés, le scénario de repos déterminé comprenant la chronologie compatible ayant le meilleur classement selon ladite relation d'ordre.

## Patentansprüche

1. System (10) der Bestimmung eines Szenarios für Ruhe im Flug einer Besatzung eines Luftfahrzeugs (12), wobei die Besatzung mindestens zwei Piloten umfasst, die geeignet sind, das Luftfahrzeug (12) während eines Fluges des Luftfahrzeugs (12) zu fliegen, wobei das System (10) umfasst:
- eine Anzeigevorrichtung (16), umfassend einen Bildschirm (20),
- ein Modul (32) zur Erfassung von Eingaben von Kontext und von Randbedingungen des Fluges,
- ein Modul (34) der Bestimmung mindestens eines Szenarios für Ruhe der Besatzung für den Flug ausgehend von den genannten erfassten Eingaben, wobei das Bestimmungsmodul (34) geeignet ist, das genannte Szenario für Ruhe ausgehend von mindestens einer Simulation durch ein biomathematisches Modell der Ermüdung zu bestimmen, das als Variablen mindestens eine der genannten erfassten Eingaben aufweist,
- ein Modul (36) für Anzeigemanagement, konfiguriert zum Anzeigen des bestimmten Szenarios für Ruhe auf dem Bildschirm (20) der Anzeigevorrichtung (16);
**dadurch gekennzeichnet, dass** das Szenario für Ruhe für eine Reiseflugphase des Fluges bestimmt wird und eine Chronologie von Ruhezeitraum/-zeiträumen umfasst, wobei jeder Zeitraum einem der Piloten zugeordnet ist, und die Eingaben von Kontext und von Randbedingungen des Fluges mindestens eine Randbedingung des Fliegens durch die Besatzung des Luftfahrzeugs (12) umfassen, wobei die oder eine der Randbedingungen des Fliegens ein Parameter ist, der für eine Toleranz gegenüber einem laufenden meteorologischen Kontext des Fluges repräsentativ ist,
wobei das Bestimmungsmodul (34) konfiguriert ist zum Bestimmen mindestens einer Chronologie von Ruhezeitraum/-zeiträumen, die mit jeder Randbedingung des Fliegens kompatibel ist,
wobei das Bestimmungsmodul (34) konfiguriert ist zum Simulieren und Bestimmen, für jede kompatible Chronologie, mindestens eines Ermüdungsniveaus jedes Piloten, durch das biomathematische Modell,
wobei das Bestimmungsmodul (34) konfiguriert ist zum Klassifizieren jeder kompatiblen Chronologie gemäß einer Ordnungsrelation, die ausgehend von den simulierten Ermüdungsniveaus festgelegt ist, wobei das bestimmte Szenario für Ruhe die kompatible Chronologie umfasst, die die beste Klassifizierung gemäß der genannten Ordnungsrelation aufweist.

2. System (10) gemäß Anspruch 1, in dem die oder eine der Randbedingungen des Fliegens eine erforderliche Mindestanzahl von Pilot(en) im Dienst zu jedem Zeitpunkt des Reisefluges ist;
und/oder die oder eine der Randbedingungen des Fliegens eine vorbestimmte zeitliche oder geografische Zone des Fluges ist, die eine vorbestimmte Anzahl von Piloten im Dienst erfordert, wobei die vorbestimmte Anzahl zum Beispiel die Gesamtheit der Piloten der Besatzung ist;
und/oder die oder eine der Randbedingungen des Fliegens eine zeitliche oder geografische Zone des Fluges ist, die einen vorbestimmten Piloten im Dienst unter den Piloten der Besatzung erfordert.

3. System (10) gemäß einem beliebigen der Ansprüche 1 oder 2, in dem die Eingaben von Kontext und von Randbedingungen des Fluges, für jeden Piloten, mindestens einen Vorflug-Ermüdungsparameter umfassen, der für einen vom Piloten deklarierten Vorflug-Ermüdungszustand repräsentativ ist, wobei das biomathematische Modell den genannten Vorflug-Ermüdungsparameter als Variable aufweist.

4. System (10) gemäß einem beliebigen der Ansprüche 1 bis 3, in dem das Bestimmungsmodul (34) konfiguriert ist zum Simulieren und Bestimmen, durch das biomathematische Modell und für jede kompatible Chronologie, eines maximalen Ermüdungsniveaus jedes Piloten im Dienst während jedes Ruhezeitraums jedes anderen Piloten und/oder eines Ermüdungsniveaus zu einem berechneten Zeitpunkt des Beginns des Sinkflugs des Luftfahrzeugs (12) von mindestens jedem Piloten, der zu dem genannten Zeitpunkt im Dienst vorgesehen ist, wobei die Ordnungsrelation auf mindestens einem der genannten simulierten und bestimmten Ermüdungsniveaus festgelegt ist.

5. System (10) gemäß einem beliebigen der Ansprüche 1 bis 4, in dem die Eingaben von Kontext und von Randbedingungen des Fluges eine verfügbare Reiseflugdauer, eine Anzahl von Ruhezeitraum/-zeiträumen, die für die Besatzung vorzusehen notwendig ist/sind, und eine Dauer des Ruhezeitraums umfassen,
wobei das Bestimmungsmodul (34) konfiguriert ist zum Diskretisieren der verfügbaren Reiseflugdauer in eine Vielzahl diskretisierter Zeitpunkte, wobei die Diskretisierung mit einem Diskretisierungsschritt durchgeführt wird,
wobei das Bestimmungsmodul (34) konfiguriert ist zum Bestimmen jeder kompatiblen Chronologie, sodass jeder Ruhezeitraum der kompatiblen Chronologie einen Anfangszeitpunkt aufweist, der einem der diskretisierten Zeitpunkte entspricht.

6. System (10) gemäß Anspruch 5, in dem das Bestimmungsmodul (34) konfiguriert ist zum Auflisten, nach Diskretisierung, aller Chronologien von Ruhezeiträumen, in denen jeder Ruhezeitraum einen Anfangszeitpunkt aufweist, der einem der diskretisierten Zeitpunkte entspricht, und zum Überprüfen, für jede aufgelistete Chronologie, ob die aufgelistete Chronologie mit jeder Randbedingung des Fliegens kompatibel ist.

7. System (10) gemäß Anspruch 6, in dem, nachdem die kompatible Chronologie bestimmt wurde, die die beste Klassifizierung aufweist, das Bestimmungsmodul (34) konfiguriert ist zum Bestimmen, für jeden Zeitraum der genannten Chronologie, eines Zeitfensters, das den Zeitraum einschließt,
wobei das Bestimmungsmodul (34) konfiguriert ist zum Diskretisieren, mit einem reduzierten Diskretisierungsschritt, jedes Zeitfensters in eine Vielzahl reduzierter diskretisierter Zeitpunkte, wobei der reduzierte Diskretisierungsschritt kleiner als der vorbestimmte Diskretisierungsschritt ist,
wobei das Bestimmungsmodul (34) konfiguriert ist zum Auflisten, nach reduzierter Diskretisierung, aller neuen Chronologien von Ruhezeiträumen, in denen jeder Ruhezeitraum einen Anfangszeitpunkt aufweist, der einem der reduzierten diskretisierten Zeitpunkte entspricht, und zum Überprüfen, für jede neue aufgelistete Chronologie, ob die neue aufgelistete Chronologie mit jeder Randbedingung des Fliegens kompatibel ist,
wobei das Bestimmungsmodul (34) konfiguriert ist zum Simulieren und Bestimmen, für jede neue kompatible Chronologie, mindestens eines neuen Ermüdungsniveaus jedes Piloten, durch das biomathematische Modell,
wobei das Bestimmungsmodul (34) konfiguriert ist zum Klassifizieren jeder neuen kompatiblen Chronologie gemäß der Ordnungsrelation, die ausgehend von den neuen simulierten Ermüdungsniveaus festgelegt ist, wobei das bestimmte Szenario für Ruhe die neue kompatible Chronologie umfasst, die die beste Klassifizierung gemäß der genannten Ordnungsrelation aufweist.

8. System (10) gemäß einem der Ansprüche 1 bis 7, in dem, vor dem Bestimmen jeder Chronologie von Ruhezeitraum/-zeiträumen, die mit jeder erfassten Randbedingung des Fliegens kompatibel ist, das Bestimmungsmodul (34) konfiguriert ist zum Überprüfen mindestens einer Kompatibilitätsbedingung zwischen mindestens zwei der erfassten Eingaben, und konfiguriert zum Modifizieren einer der erfassten Eingaben, wenn die Kompatibilitätsbedingung zwischen Eingaben nicht verifiziert ist, und/oder zum Erzeugen einer Warnung für die Besatzung, wenn die Kompatibilitätsbedingung zwischen Eingaben nicht verifiziert ist, wobei das Modul für Anzeigemanagement (36) konfiguriert ist zum Erzeugen einer grafischen Darstellung der Warnung.

9. System (10) gemäß Anspruch 8, in dem die Eingaben von Kontext und von Randbedingungen des Fluges eine verfügbare Reiseflugdauer, eine Anzahl von Ruhezeitraum/- zeiträumen, die für die Besatzung vorzusehen notwendig ist/sind, und eine Dauer des Ruhezeitraums umfassen; wobei das Bestimmungsmodul (34) konfiguriert ist zum Überprüfen einer Kompatibilitätsbedingung zwischen der verfügbaren Reiseflugdauer und den vorzusehenden Ruhezeiträumen, wobei die Kompatibilitätsbedingung ist, dass die verfügbare Reiseflugdauer ausreichend lang ist, um jeden Ruhezeitraum einzuschließen,
wobei das Bestimmungsmodul (34) vorzugsweise konfiguriert ist zum Erzeugen einer Warnung für die Besatzung, wenn die verfügbare Reiseflugdauer zu kurz ist, um jeden Ruhezeitraum einzuschließen, und/oder konfiguriert zum Reduzieren der Dauer mindestens eines der vorzusehenden Ruhezeiträume, wenn die verfügbare Reiseflugdauer zu kurz ist, um jeden Ruhezeitraum einzuschließen.

10. System (10) gemäß einem beliebigen der Ansprüche 1 bis 9, in dem jeder Ruhezeitraum eine Dauer autorisierten Schlafs und eine Dauer des Arbeitsplatzwechsels umfasst.

11. System (10) gemäß einem beliebigen der Ansprüche 1 bis 10, in dem das bestimmte Szenario für Ruhe auch, für jeden Piloten, Dienstzeiträume umfasst, wobei das Bestimmungsmodul (34) konfiguriert ist zum Simulieren und Bestimmen, durch das biomathematische Modell, einer Entwicklung eines Ermüdungsniveaus jedes Piloten im Laufe jedes Dienstzeitraums des Piloten, wobei das Modul für Anzeigemanagement (36) konfiguriert ist zum Anzeigen, auf dem Bildschirm (20) der Anzeigevorrichtung (16), einer spezifischen grafischen Darstellung des bestimmten Szenarios für Ruhe, die für jede Entwicklung repräsentativ ist.

12. System (10) gemäß einem beliebigen der vorhergehenden Ansprüche, in dem das Bestimmungssystem (10) eine Mensch-Maschine-Schnittstelle (18) umfasst und das Modul für Anzeigemanagement (36) konfiguriert ist zum Anzeigen, auf dem Bildschirm (20) der Anzeigevorrichtung (16), eines Fensters (50), umfassend eine Vielzahl grafischer Zonen zur Erfassung von Eingaben, wobei jede grafische Zone zur Erfassung von Eingaben mindestens einer der Eingaben von Kontext und von Randbedingungen des Fluges zugeordnet ist, wobei das Erfassungsmodul (32) konfiguriert ist zum Erfassen mindestens eines Teils der Eingaben von Kontext und von Randbedingungen des Fluges durch die Betätigung grafischer Elemente, die in jeder Erfassungszone angezeigt werden, wobei die Betätigung über die Mensch-Maschine-Schnittstelle (18) umgesetzt wird.

13. System (10) gemäß einem beliebigen der vorhergehenden Ansprüche, in dem das Bestimmungssystem (10) eine Mensch-Maschine-Schnittstelle (18) umfasst und das genannte Modul für Anzeigemanagement (36) konfiguriert ist zum Detektieren einer Aktion zur Modifikation des angezeigten Szenarios für Ruhe, die durch einen Bediener über die Mensch-Maschine-Schnittstelle (18) umgesetzt wird,
wobei das Bestimmungsmodul (34) geeignet ist, ein Ermüdungsniveau jedes Piloten ausgehend von dem biomathematischen Modell der Ermüdung in dem modifizierten Szenario für Ruhe zu simulieren und zu bestimmen, wobei das Modul für Anzeigemanagement (36) konfiguriert ist zum Anzeigen des modifizierten Szenarios für Ruhe, gemäß einer spezifischen grafischen Darstellung.

14. Verfahren (200) der Bestimmung eines Szenarios für Ruhe im Flug einer Besatzung eines Luftfahrzeugs (12), wobei die Besatzung mindestens zwei Piloten umfasst, die geeignet sind, das Luftfahrzeug (12) während eines Fluges des Luftfahrzeugs (12) zu fliegen, wobei das Verfahren (200) die folgenden Schritte umfasst:
- Erfassung (202) von Eingaben von Kontext und von Randbedingungen des Fluges,
- Bestimmung (206) mindestens eines Szenarios für Ruhe der Besatzung für den Flug ausgehend von den genannten erfassten Eingaben, wobei das genannte Szenario für Ruhe ausgehend von mindestens einer Simulation durch ein biomathematisches Modell der Ermüdung bestimmt wird, das als Variablen mindestens eine der genannten erfassten Eingaben aufweist, und
- Anzeige des bestimmten Szenarios für Ruhe auf einem Bildschirm (20) einer Anzeigevorrichtung (16);
**dadurch gekennzeichnet, dass** das Szenario für Ruhe für eine Reiseflugphase des Fluges bestimmt wird und eine Chronologie von Ruhezeitraum/-zeiträumen umfasst, wobei jeder Zeitraum einem der Piloten zugeordnet ist, und die Eingaben von Kontext und von Randbedingungen des Fluges mindestens eine Randbedingung des Fliegens durch die Besatzung des Luftfahrzeugs (12) umfassen, wobei die oder eine der Randbedingungen des Fliegens ein für eine Toleranz gegenüber einem aktuellen meteorologischen Kontext des Fluges repräsentativer Parameter ist,
wobei die Bestimmung (206) umfasst:
* die Bestimmung mindestens einer Chronologie von Ruhezeitraum/-zeiträumen, die mit jeder Randbedingung des Fliegens kompatibel ist,
* die Simulation und die Bestimmung, für jede kompatible Chronologie, mindestens eines Ermüdungsniveaus jedes Piloten durch das biomathematische Modell,
* die Einstufung jeder kompatiblen Chronologie gemäß einer Ordnungsrelation, die ausgehend von den simulierten Ermüdungsniveaus erstellt wird, wobei das bestimmte Szenario für Ruhe die kompatible Chronologie umfasst, die die beste Einstufung gemäß der genannten Ordnungsrelation aufweist.

## Claims

1. A system (10) for determining an in-flight rest scenario for a crew of an aircraft (12), the crew comprising at least two pilots able to fly the aircraft (12) during a flight of the aircraft (12), the system (10) comprising:
- a display device (16) comprising a screen (20),
- a module (32) for acquiring flight context and constraints inputs,
- a module (34) for determining at least one in-flight rest scenario for the crew from said acquired inputs, the determination module (34) being able to determine said rest scenario from at least one simulation by a biomathematical fatigue model having at least one of said acquired inputs as variables,
- a display management module (36), configured to display the determined rest scenario on the screen (20) of the display device (16);
**characterized in that** the rest scenario is determined for a cruise phase of flight and comprises a timeline of rest period(s), each rest period being assigned to one of the pilots, and the flight context and constraints inputs comprise at least one constraint on piloting by the crew of the aircraft (12),
the determination module (34) being configured to determine at least one timeline of rest period(s) compatible with each piloting constraint,
the determination module (34) being configured to simulate and determine at least one fatigue level of each pilot, for each compatible timeline, by the biomathematical model,
the determination module (34) being configured to classify each compatible timeline according to an order relationship established from the simulated fatigue levels, the determined rest scenario comprising the compatible timeline that has the best classification according to said order relationship.

2. The system (10) according to claim 1, wherein the or one of the piloting constraints is a minimum number of pilot(s) required on duty at each moment of the cruise;
and/or the or one of the piloting constraints is a predetermined temporal or geographical area of flight requiring a predetermined number of pilots on duty, the predetermined number being for example all of the pilots in the crew;
and/or the or one of the piloting constraints is a temporal or geographical area of flight requiring a predetermined pilot on duty from among the pilots of the crew;
and/or the or one of the piloting constraints is a parameter showing a tolerance to a current meteorological flight context.

3. The system (10) according to any one of claims 1 or 2, wherein the flight context and constraints inputs include at least one pre-flight fatigue parameter for each pilot, representing a pre-flight fatigue state reported by the pilot, the biomathematical model having said pre-flight fatigue parameter as a variable.

4. The system (10) according to any one of claims 1 to 3, wherein the determination module (34) is configured to simulate and determine, by the biomathematical model and for each compatible timeline, a maximum fatigue level of each pilot on duty during each rest period of each other pilot and/or a fatigue level at a calculated top of descent time of the aircraft (12) of at least each pilot scheduled to be on duty at said time, the order relationship being established on at least one of said simulated and determined fatigue levels.

5. The system (10) according to any one of claims 1 to 4, wherein the flight context and constraint inputs include an available cruise time, a number of required rest period(s) to be provided for the crew, and a rest period duration,
the determination module (34) being configured to discretize the available cruise time into a plurality of discretized moments, the discretization being done with a predetermined discretized timestep,
the determination module (34) being configured to determine each compatible timeline such that each rest period of the compatible timeline has a start time corresponding to one of the discretized moments.

6. The system (10) according to claim 5, wherein the determination module (34) is configured, after discretization, to list all of the timelines of rest periods in which each rest period has a start time corresponding to one of the discretized moments, and to verify, for each listed timeline, whether the listed timeline is compatible with each piloting constraint.

7. The system (10) according to claim 6, wherein, after determining the compatible timeline having the best classification, the determination module (34) is configured to determine, for each rest period of said timeline, a time window including the rest period,
the determination module (34) being configured to discretize, with a reduced discretized timestep, each time window into a plurality of reduced discretized moments, the reduced discretized timestep being smaller than the predetermined discretized timestep,
the determination module (34) being configured, after reduced discretization, to list all the new timelines of rest periods in which each rest period has a start time corresponding to one of the reduced discretized moments, and to verify, for each listed new timeline, whether the listed new timeline is compatible with each piloting constraint,
the determination module (34) being configured to simulate and determine, by the biomathematical model and for each new compatible timeline, at least one new fatigue level of each pilot,
the determination module (34) being configured to classify each new compatible timeline, according to the order relationship, established from the new simulated fatigue levels, the determined rest scenario comprising the new compatible timeline having the best classification according to said order relationship.

8. The system (10) according to claim 1 to 7, wherein, before determining each timeline of rest period(s) compatible with each acquired piloting constraint, the determination module (34) is configured to verify at least one compliance condition between at least two of the acquired inputs, and configured to modify one of the acquired inputs if the compliance condition between inputs is not verified and/or generate an alert for the crew if the compliance condition between inputs is not verified, the display management module (36) being configured to generate a graphical representation of the alert.

9. The system (10) according to claim 8, wherein the flight context and constraints inputs include an available cruise time, a number of required rest period(s) to be scheduled for the crew, and a rest period duration; the determination module (34) being configured to verify a compliance condition between the available cruise time and the rest periods to be scheduled, the compliance condition being that the available cruise time is long enough to include each rest period,
the determination module (34) preferably being configured to generate an alert to the crew if the available cruise time is too short to include each rest period and/or configured to reduce the duration of at least one of the scheduled rest periods if the available cruise time is too short to include each rest period.

10. The system (10) according to any one of claims 1 to 9, wherein each rest period includes an authorized sleep duration and a shift transition duration.

11. The system (10) according to any one of claims 1 to 10, wherein the determined rest scenario also includes duty periods for each pilot, the determination module (34) being configured to simulate and determine, by the biomathematical model, each pilot's fatigue level evolution over each pilot's duty period, the display management module (36) being configured to display a specific graphical representation of the determined rest scenario representing each evolution, on the screen (20) of the display device (16).

12. The system (10) according to any one of the preceding claims, wherein the determination system (10) comprises a human/machine interface (18) and the display management module (36) is configured to display a window (50) on the screen (20) of the display device (16), comprising a plurality of graphical input acquisition areas, each graphical input acquisition areas being associated to at least one of the flight context and constraints inputs, the acquisition module (32) being configured to acquire at least part of the flight context and constraints inputs by activating graphical elements displayed in each acquisition area, the actuation being implemented via the human/machine interface (18).

13. The system (10) according to any of the preceding claims, wherein the determination system (10) comprises a human/machine interface (18) and said display management module (36) is configured to detect an action of modification of the displayed rest scenario implemented by an operator through the human/machine interface (18),
the determination module (34) being able to simulate and determine each pilot's fatigue level from the biomathematical fatigue model in the modified rest scenario, the display management module (36) being configured to display the modified rest scenario, according to a specific graphical representation.

14. A method (200) for determining an in-flight rest scenario for a crew of an aircraft (12), the crew comprising at least two pilots able to fly the aircraft (12) during a flight of the aircraft (12), the method (200) comprising the following steps:
- acquiring (202) flight context and constraints inputs,
- determining (206) at least one in-flight rest scenario for the crew from said acquired inputs, said rest scenario being determined from at least one simulation by a biomathematical fatigue model, having at least one of said acquired inputs as variables, and
- displaying the determined rest scenario on a screen (20) of a display device (16).
**characterized in that** the rest scenario is determined for a cruise phase of flight and comprises a timeline of rest period(s), each rest period being assigned to one of the pilots, and the flight context and constraints inputs comprise at least one constraint on piloting by the crew of the aircraft (12),
wherein determining (206) comprises:
* determining at least one timeline of rest period(s) compatible with each piloting constraint,
* simulating and determining at least one fatigue level of each pilot, for each compatible timeline, by the biomathematical model,
* classifying each compatible timeline according to an order relationship established from the simulated fatigue levels, the determined rest scenario comprising the compatible timeline that has the best classification according to said order relationship.
